# EUROPEAN PATENT SPECIFICATION

(11) **EP 1 363 930 B1**
(45) Date of publication and mention of the grant of the patent: **16.08.2006**
(21) Application number: 00978578.3
(22) Date of filing: 14.11.2000
(51) Int. Cl.: C07K 14/47, A61K 39/00, C12N 15/00, C12N 15/63, C12N 15/85

(54) **A METHOD FOR GENERATING GENETICALLY ALTERED ANTIGENS**
VERFAHREN ZUR ERZEUGUNG VON GENETISCH VERÄNDERTEN ANTIGENEN
METHODE PERMETTANT DE PRODUIRE DES ANTIGENES GENETIQUEMENT MODIFIES

(43) Date of publication of application: 26.11.2003
(73) Proprietor: Morphotek Inc., Exton, PA 19341 (US)
(72) Inventor: NICOLAIDES, Nicholas, C., Boothwyn, PA 19061 (US); GRASSO, Luigi, Bala Cynwyd, PA 19004 (US); SASS, Philip, M., Audubon, PA 19403 (US)
(74) Representative: W.P. Thompson & Co.
(86) International application number: PCT/US2000/031135
(87) International publication number: WO 2002/040499

(56) References cited:
- WO-A1-97/08312
- CA-A- 2 240 609
- US-A- 6 146 894
- NICOLAIDES ET AL.: 'Mutation of two PMS homologues in hereditary nonpolyposis colon cancer' NATURE vol. 371, 01 September 1994, pages 75 - 80, XP002936633
- PAPADOPOULOS ET AL.: 'Mutation of a mutL homolog in hereditary colon cancer' SCIENCE vol. 263, 18 March 1994, pages 1625 - 1629, XP002936635
- NICOLAIDES ET AL.: 'A naturally occurring hPMS2 mutation can confer a dominant negative mutator phenotype' MOLEC. CELL. BIOL. vol. 18, no. 3, March 1998, pages 1635 - 1641, XP002936634

### SEQUENCE LISTING

<110> Nicolaides, Nicholas C
   Grasso, Luigi
   Sass, Philip M
<120> METHODS FOR GENERATING GENETICALLY ALTERED ANTIGENS
<130> MOR-0016
<140> 00/000,000
   <141> 2000-11-14
<160> 17
<170> PatentIn Ver. 2.1
<210> 1
   <211> 86
   <212> DNA
   <213> Artificial Sequence
<220>
   <223> Description of Artificial Sequence:sense primer
<400> 1
   aagcttccat gtacaggatg caactcctgt cttgcattgc actaagtctt gcacttgtca 60
   caaacagtgc acaaaagctg gagctc 86
<210> 2
   <211> 51
   <212> DNA
   <213> Artificial Sequence
<220>
   <223> Description of Artificial Sequence:antisense primer
<400> 2
   ccggatccct actagtggtg atggtgatgg tggcttgata tcgaattcct g 51
<210> 3
   <211> 22
   <212> DNA
   <213> Artificial Sequence
<220>
   <223> Description of Artificial Sequence:sense primer
<400> 3
   catgtacagg atgcaactcc tg 22
<210> 4
   <211> 23
   <212> DNA
   <213> Artificial Sequence
<220>
   <223> Description of Artificial Sequence:antisense primer
<400> 4
   tactagtggt gatggtgatg gtg 23
<210> 5
   <211> 859
   <212> PRT
   <213> Mus musculus
<400> 5
   Met Glu Gln Thr Glu Gly Val Ser Thr Glu Cys Ala Lys Ala Ile Lys
   1               5                   10                  15
   Pro Ile Asp Gly Lys Ser Val His Gln Ile Cys Ser Gly Gln Val Ile
               20                  25                  30
   Leu Ser Leu Ser Thr Ala Val Lys Glu Leu Ile Glu Asn Ser Val Asp
           35                  40                  45
   Ala Gly Ala Thr Thr Ile Asp Leu Arg Leu Lys Asp Tyr Gly Val Asp
       50                  55                  60
   Leu Ile Glu Val Ser Asp Asn Gly Cys Gly Val Glu Glu Glu Asn Phe
   65                  70                  75                  80
   Glu Gly Leu Ala Leu Lys His His Thr Ser Lys Ile Gln Glu Phe Ala
                   85                  90                  95
   Asp Leu Thr Gln Val Glu Thr Phe Gly Phe Arg Gly Glu Ala Leu Ser
               100                 105                 110
   Ser Leu Cys Ala Leu Ser Asp Val Thr Ile Ser Thr Cys His Gly Ser
   115 120 125
   Ala Ser Val Gly Thr Arg Leu Val Phe Asp His Asn Gly Lys Ile Thr
   130 135 140
   Gln Lys Thr Pro Tyr Pro Arg Pro Lys Gly Thr Thr Val Ser Val Gln
   145 150 155 160
   His Leu Phe Tyr Thr Leu Pro Val Arg Tyr Lys Glu Phe Gln Arg Asn
   165 170 175
   Ile Lys Lys Glu Tyr Ser Lys Met Val Gln Val Leu Gln Ala Tyr Cys
   180 185 190
   Ile Ile Ser Ala Gly Val Arg Val Ser Cys Thr Asn Gln Leu Gly Gln
   195 200 205
   Gly Lys Arg His Ala Val Val Cys Thr Ser Gly Thr Ser Gly Met Lys
   210 215 220
   Glu Asn Ile Gly Ser Val Phe Gly Gln Lys Gln Leu Gln Ser Leu Ile
   225 230 235 240
   Pro Phe Val Gln Leu Pro Pro Ser Asp Ala Val Cys Glu Glu Tyr Gly
   245 250 255
   Leu Ser Thr Ser Gly Arg His Lys Thr Phe Ser Thr Phe Arg Ala Ser
   260 265 270
   Phe His Ser Ala Arg Thr Ala Pro Gly Gly Val Gln Gln Thr Gly Ser
   275 280 285
   Phe Ser Ser Ser Ile Arg Gly Pro Val Thr Gln Gln Arg Ser Leu Ser
   290 295 300
   Leu Ser Met Arg Phe Tyr His Met Tyr Asn Arg His Gln Tyr Pro Phe
   305 310 315 320
   Val Val Leu Asn Val Ser Val Asp Ser Glu Cys Val Asp Ile Asn Val
   325 330 335
   Thr Pro Asp Lys Arg Gln Ile Leu Leu Gln Glu Glu Lys Leu Leu Leu
   340 345 350
   Ala Val Leu Lys Thr Ser Leu Ile Gly Met Phe Asp Ser Asp Ala Asn
   355 360 365
   Lys Leu Asn Val Asn Gln Gln Pro Leu Leu Asp Val Glu Gly Asn Leu
   370 375 380
   Val Lys Leu His Thr Ala Glu Leu Glu Lys Pro Val Pro Gly Lys Gln
   385 390 395 400
   Asp Asn Ser Pro Ser Leu Lys Ser Thr Ala Asp Glu Lys Arg Val Ala
   405 410 415
   Ser Ile Ser Arg Leu Arg Glu Ala Phe Ser Leu His Pro Thr Lys Glu
   420 425 430
   Ile Lys Ser Arg Gly Pro Glu Thr Ala Glu Leu Thr Arg Ser Phe Pro
   435 440 445
   Ser Glu Lys Arg Gly Val Leu Ser Ser Tyr Pro Ser Asp Val Ile Ser
   450 455 460
   Tyr Arg Gly Leu Arg Gly Ser Gln Asp Lys Leu Val Ser Pro Thr Asp
   465 470 475 480
   Ser Pro Gly Asp Cys Met Asp Arg Glu Lys Ile Glu Lys Asp Ser Gly
   485 490 495
   Leu Ser Ser Thr Ser Ala Gly Ser Glu Glu Glu Phe Ser Thr Pro Glu
   500 505 510
   Val Ala Ser Ser Phe Ser Ser Asp Tyr Asn Val Ser Ser Leu Glu Asp
   515 520 525
   Arg Pro Ser Gln Glu Thr Ile Asn Cys Gly Asp Leu Asp Cys Arg Pro
   530 535 540
   Pro Gly Thr Gly Gln Ser Leu Lys Pro Glu Asp His Gly Tyr Gln Cys
   545 550 555 560
   Lys Ala Leu Pro Leu Ala Arg Leu Ser Pro Thr Asn Ala Lys Arg Phe
   565 570 575
   Lys Thr Glu Glu Arg Pro Ser Asn Val Asn Ile Ser Gln Arg Leu Pro
   580 585 590
   Gly Pro Gln Ser Thr Ser Ala Ala Glu Val Asp Val Ala Ile Lys Met
   595 600 605
   Asn Lys Arg Ile Val Leu Leu Glu Phe Ser Leu Ser Ser Leu Ala Lys
   610 615 620
   Arg Met Lys Gln Leu Gln His Leu Lys Ala Gln Asn Lys His Glu Leu
   625                 630                 635                 640
   Ser Tyr Arg Lys Phe Arg Ala Lys Ile Cys Pro Gly Glu Asn Gln Ala
                   645                 650                 655
   Ala Glu Asp Glu Leu Arg Lys Glu Ile Ser Lys Ser Met Phe Ala Glu
               660                 665                 670
   Met Glu Ile Leu Gly Gln Phe Asn Leu Gly Phe Ile Val Thr Lys Leu
           675                 680                 685
   Lys Glu Asp Leu Phe Leu Val Asp Gln His Ala Ala Asp Glu Lys Tyr
       690                 695                 700
   Asn Phe Glu Met Leu Gln Gln His Thr Val Leu Gln Ala Gln Arg Leu
   705                 710                 715                 720
   Ile Thr Pro Gln Thr Leu Asn Leu Thr Ala Val Asn Glu Ala Val Leu
                   725                 730                 735
   Ile Glu Asn Leu Glu Ile Phe Arg Lys Asn Gly Phe Asp Phe Val Ile
               740                 745                 750
   Asp Glu Asp Ala Pro Val Thr Glu Arg Ala Lys Leu Ile Ser Leu Pro
           755                 760                 765
   Thr Ser Lys Asn Trp Thr Phe Gly Pro Gln Asp Ile Asp Glu Leu Ile
       770                 775                 780
   Phe Met Leu Ser Asp Ser Pro Gly Val Met Cys Arg Pro Ser Arg Val
   785                 790                 795                 800
   Arg Gln Met Phe Ala Ser Arg Ala Cys Arg Lys Ser Val Met Ile Gly
                   805                 810                 815
   Thr Ala Leu Asn Ala Ser Glu Met Lys Lys Leu Ile Thr His Met Gly
               820                 825                 830
   Glu Met Asp His Pro Trp Asn Cys Pro His Gly Arg Pro Thr Met Arg
           835                 840                 845
   His Val Ala Asn Leu Asp Val Ile Ser Gln Asn
       850                 855
<210> 6
   <211> 3056
   <212> DNA
   <213> Mus musculus
<400> 6
   gaattccggt gaaggtcctg aagaatttcc agattcctga gtatcattgg aggagacaga 60
   taacctgtcg tcaggtaacg atggtgtata tgcaacagaa atgggtgttc ctggagacgc 120
   gtcttttccc gagagcggca ccgcaactct cccgcggtga ctgtgactgg aggagtcctg 180
   catccatgga gcaaaccgaa ggcgtgagta cagaatgtgc taaggccatc aagcctattg 240
   atgggaagtc agtccatcaa atttgttctg ggcaggtgat actcagttta agcaccgctg 300
   tgaaggagtt gatagaaaat agtgtagatg ctggtgctac tactattgat ctaaggctta 360
   aagactatgg ggtggacctc attgaagttt cagacaatgg atgtggggta gaagaagaaa 420
   actttgaagg tctagctctg aaacatcaca catctaagat tcaagagttt gccgacctca 480
   cgcaggttga aactttcggc tttcgggggg aagctctgag ctctctgtgt gcactaagtg 540
   atgtcactat atctacctgc cacgggtctg caagcgttgg gactcgactg gtgtttgacc 600
   ataatgggaa aatcacccag aaaactccct acccccgacc taaaggaacc acagtcagtg 660
   tgcagcactt attttataca ctacccgtgc gttacaaaga gtttcagagg aacattaaaa 720
   aggagtattc caaaatggtg caggtcttac aggcgtactg tatcatctca gcaggcgtcc 780
   gtgtaagctg cactaatcag ctcggacagg ggaagcggca cgctgtggtg tgcacaagcg 840
   gcacgtctgg catgaaggaa aatatcgggt ctgtgtttgg ccagaagcag ttgcaaagcc 900
   tcattccttt tgttcagctg ccccctagtg acgctgtgtg tgaagagtac ggcctgagca 960
   cttcaggacg ccacaaaacc ttttctacgt ttcgggcttc atttcacagt gcacgcacgg 1020
   cgccgggagg agtgcaacag acaggcagtt tttcttcatc aatcagaggc cctgtgaccc 1080
   agcaaaggtc tctaagcttg tcaatgaggt tttatcacat gtataaccgg catcagtacc 1140
   catttgtcgt ccttaacgtt tccgttgact cagaatgtgt ggatattaat gtaactccag 1200
   ataaaaggca aattctacta caagaagaga agctattgct ggccgtttta aagacctcct 1260
   tgataggaat gtttgacagt gatgcaaaca agcttaatgt caaccagcag ccactgctag 1320
   atgttgaagg taacttagta aagctgcata ctgcagaact agaaaagcct gtgccaggaa 1380
   agcaagataa ctctccttca ctgaagagca cagcagacga gaaaagggta gcatccatct 1440
   ccaggctgag agaggccttt tctcttcatc ctactaaaga gatcaagtct aggggtccag 1500
   agactgctga actgacacgg agttttccaa gtgagaaaag ggcgtgttta tcctcttatc 1560
   cttcagacgt catctcttac agaggcctcc gtggctcgca ggacaaattg gtgagtccca 1620
   cggacagccc tggtgactgt atggacagag agaaaataga aaaagactca gggctcagca 1680
   gcacctcagc tggctctgag gaagagttca gcaccccaga agtggccagt agctttagca 1740
   gtgactataa cgtgagctcc ctagaagaca gaccttctca ggaaaccata aactgtggtg 1800
   acctggactg ccgtcctcca ggtacaggac agtccttgaa gccagaagac catggatatc 1860
   aatgcaaagc tctacctcta gctcgtctgt cacccacaaa tgccaagcgc ttcaagacag 1920
   aggaaagacc ctcaaatgtc aacatttctc aaagattgcc tggtcctcag agcacctcag 1980
   cagctgaggt cgatgtagcc ataaaaatga ataagagaat cgtgctcctc gagttctctc 2040
   tgagttctct agctaagcga atgaagcagt tacagcacct aaaggcgcag aacaaacatg 2100
   aactgagtta cagaaaattt agggccaaga tttgccctgg agaaaaccaa gcagcagaag 2160
   atgaactcag aaaagagatt agtaaatcga tgtttgcaga gatggagatc ttgggtcagt 2220
   ttaacctggg atttatagta accaaactga aagaggacct cttcctggtg gaccagcatg 2280
   ctgcggatga gaagtacaac tttgagatgc tgcagcagca cacggtgctc caggcgcaga 2340
   ggctcatcac accccagact ctgaacttaa ctgctgtcaa tgaagctgta ctgatagaaa 2400
   atctggaaat attcagaaag aatggctttg actttgtcat tgatgaggat gctccagtca 2460
   ctgaaagggc taaattgatt tccttaccaa ctagtaaaaa ctggaccttt ggaccccaag 2520
   atatagatga actgatcttt atgttaagtg acagccctgg ggtcatgtgc cggccctcac 2580
   gagtcagaca gatgtttgct tccagagcct gtcggaagtc agtgatgatt ggaacggcgc 2640
   tcaatgcgag cgagatgaag aagctcatca cccacatggg tgagatggac cacccctgga 2700
   actgccccca cggcaggcca accatgaggc acgttgccaa tctggatgtc atctctcaga 2760
   actgacacac cccttgtagc atagagttta ttacagattg ttcggtttgc aaagagaagg 2820
   ttttaagtaa tctgattatc gttgtacaaa aattagcatg ctgctttaat gtactggatc 2880
   catttaaaag cagtgttaag gcaggcatga tggagtgttc ctctagctca gctacttggg 2940
   tgatccggtg ggagctcatg tgagcccagg actttgagac cactccgagc cacattcatg 3000
   agactcaatt caaggacaaa aaaaaaaaga tatttttgaa gccttttaaa aaaaaa     3056
<210> 7
   <211> 862
   <212> PRT
   <213> Homo sapiens
<400> 7
   Met Glu Arg Ala Glu Ser Ser Ser Thr Glu Pro Ala Lys Ala Ile Lys
    1               5                  10                  15
   Pro Ile Asp Arg Lys Ser Val His Gln Ile Cys Ser Gly Gln Val Val
               20                  25                  30
   Leu Ser Leu Ser Thr Ala Val Lys Glu Leu Val Glu Asn Ser Leu Asp
           35                  40                  45
   Ala Gly Ala Thr Asn Ile Asp Leu Lys Leu Lys Asp Tyr Gly Val Asp
       50                  55                  60
   Leu Ile Glu Val Ser Asp Asn Gly Cys Gly Val Glu Glu Glu Asn Phe
   65                  70                  75                  80
   Glu Gly Leu Thr Leu Lys His His Thr Ser Lys Ile Gln Glu Phe Ala
                   85                  90                  95
   Asp Leu Thr Gln Val Glu Thr Phe Gly Phe Arg Gly Glu Ala Leu Ser
               100                 105                 110
   Ser Leu Cys Ala Leu Ser Asp Val Thr Ile Ser Thr Cys His Ala Ser
           115                 120                 125
   Ala Lys Val Gly Thr Arg Leu Met Phe Asp His Asn Gly Lys Ile Ile
       130                 135                 140
   Gln Lys Thr Pro Tyr Pro Arg Pro Arg Gly Thr Thr Val Ser Val Gln
   145                 150                 155                 160
   Gln Leu Phe Ser Thr Leu Pro Val Arg His Lys Glu Phe Gln Arg Asn
                   165                 170                 175
   Ile Lys Lys Glu Tyr Ala Lys Met Val Gln Val Leu His Ala Tyr Cys
   180 185 190
   Ile Ile Ser Ala Gly Ile Arg Val Ser Cys Thr Asn Gln Leu Gly Gln
   195 200 205
   Gly Lys Arg Gln Pro Val Val Cys Thr Gly Gly Ser Pro Ser Ile Lys
   210 215 220
   Glu Asn Ile Gly Ser Val Phe Gly Gln Lys Gln Leu Gln Ser Leu Ile
   225 230 235 240
   Pro Phe Val Gln Leu Pro Pro Ser Asp Ser Val Cys Glu Glu Tyr Gly
   245 250 255
   Leu Ser Cys Ser Asp Ala Leu His Asn Leu Phe Tyr Ile Ser Gly Phe
   260 265 270
   Ile Ser Gln Cys Thr His Gly Val Gly Arg Ser Ser Thr Asp Arg Gln
   275 280 285
   Phe Phe Phe Ile Asn Arg Arg Pro Cys Asp Pro Ala Lys Val Cys Arg
   290 295 300
   Leu Val Asn Glu Val Tyr His Met Tyr Asn Arg His Gln Tyr Pro Phe
   305 310 315 320
   Val Val Leu Asn Ile Ser Val Asp Ser Glu Cys Val Asp Ile Asn Val
   325 330 335
   Thr Pro Asp Lys Arg Gln Ile Leu Leu Gln Glu Glu Lys Leu Leu Leu
   340 345 350
   Ala Val Leu Lys Thr Ser Leu Ile Gly Met Phe Asp Ser Asp Val Asn
   355 360 365
   Lys Leu Asn Val Ser Gln Gln Pro Leu Leu Asp Val Glu Gly Asn Leu
   370 375 380
   Ile Lys Met His Ala Ala Asp Leu Glu Lys Pro Met Val Glu Lys Gln
   385 390 395 400
   Asp Gln Ser Pro Ser Leu Arg Thr Gly Glu Glu Lys Lys Asp Val Ser
   405 410 415
   Ile Ser Arg Leu Arg Glu Ala Phe Ser Leu Arg His Thr Thr Glu Asn
   420 425 430
   Lys Pro His Ser Pro Lys Thr Pro Glu Pro Arg Arg Ser Pro Leu Gly
   435 440 445
   Gln Lys Arg Gly Met Leu Ser Ser Ser Thr Ser Gly Ala Ile Ser Asp
   450 455 460
   Lys Gly Val Leu Arg Pro Gln Lys Glu Ala Val Ser Ser Ser His Gly
   465 470 475 480
   Pro Ser Asp Pro Thr Asp Arg Ala Glu Val Glu Lys Asp Ser Gly His
   485 490 495
   Gly Ser Thr Ser Val Asp Ser Glu Gly Phe Ser Ile Pro Asp Thr Gly
   500 505 510
   Ser His Cys Ser Ser Glu Tyr Ala Ala Ser Ser Pro Gly Asp Arg Gly
   515 520 525
   Ser Gln Glu His Val Asp Ser Gln Glu Lys Ala Pro Glu Thr Asp Asp
   530 535 540
   Ser Phe Ser Asp Val Asp Cys His Ser Asn Gln Glu Asp Thr Gly Cys
   545 550 555 560
   Lys Phe Arg Val Leu Pro Gln Pro Thr Asn Leu Ala Thr Pro Asn Thr
   565 570 575
   Lys Arg Phe Lys Lys Glu Glu Ile Leu Ser Ser Ser Asp Ile Cys Gln
   580 585 590
   Lys Leu Val Asn Thr Gln Asp Met Ser Ala Ser Gln Val Asp Val Ala
   595 600 605
   Val Lys Ile Asn Lys Lys Val Val Pro Leu Asp Phe Ser Met Ser Ser
   610 615 620
   Leu Ala Lys Arg Ile Lys Gln Leu His His Glu Ala Gln Gln Ser Glu
   625 630 635 640
   Gly Glu Gln Asn Tyr Arg Lys Phe Arg Ala Lys Ile Cys Pro Gly Glu
   645 650 655
   Asn Gln Ala Ala Glu Asp Glu Leu Arg Lys Glu Ile Ser Lys Thr Met
   660 665 670
   Phe Ala Glu Met Glu Ile Ile Gly Gln Phe Asn Leu Gly Phe Ile Ile
   675 680 685
   Thr Lys Leu Asn Glu Asp Ile Phe Ile Val Asp Gln His Ala Thr Asp
   690 695 700
   Glu Lys Tyr Asn Phe Glu Met Leu Gln Gln His Thr Val Leu Gln Gly
   705 710 715 720
   Gln Arg Leu Ile Ala Pro Gln Thr Leu Asn Leu Thr Ala Val Asn Glu
   725 730 735
   Ala Val Leu Ile Glu Asn Leu Glu Ile Phe Arg Lys Asn Gly Phe Asp
   740 745 750
   Phe Val Ile Asp Glu Asn Ala Pro Val Thr Glu Arg Ala Lys Leu Ile
   755 760 765
   Ser Leu Pro Thr Ser Lys Asn Trp Thr Phe Gly Pro Gln Asp Val Asp
   770 775 780
   Glu Leu Ile Phe Met Leu Ser Asp Ser Pro Gly Val Met Cys Arg Pro
   785 790 795 800
   Ser Arg Val Lys Gln Met Phe Ala Ser Arg Ala Cys Arg Lys Ser Val
   805 810 815
   Met Ile Gly Thr Ala Leu Asn Thr Ser Glu Met Lys Lys Leu Ile Thr
   820 825 830
   His Met Gly Glu Met Asp His Pro Trp Asn Cys Pro His Gly Arg Pro
   835 840 845
   Thr Met Arg His Ile Ala Asn Leu Gly Val Ile Ser Gln Asn
   850 855 860
<210> 8
   <211> 2771
   <212> DNA
   <213> Homo sapiens
<400> 8
   cgaggcggat cgggtgttgc atccatggag cgagctgaga gctcgagtac agaacctgct 60
   aaggccatca aacctattga tcggaagtca gtccatcaga tttgctctgg gcaggtggta 120
   ctgagtctaa gcactgcggt aaaggagtta gtagaaaaca gtctggatgc tggtgccact 180
   aatattgatc taaagcttaa ggactatgga gtggatctta ttgaagtttc agacaatgga 240
   tgtggggtag aagaagaaaa cttcgaaggc ttaactctga aacatcacac atctaagatt 300
   caagagtttg ccgacctaac tcaggttgaa acttttggct ttcgggggga agctctgagc 360
   tcactttgtg cactgagcga tgtcaccatt tctacctgcc acgcatcggc gaaggttgga 420
   actcgactga tgtttgatca caatgggaaa attatccaga aaacccccta cccccgcccc 480
   agagggacca cagtcagcgt gcagcagtta ttttccacac tacctgtgcg ccataaggaa 540
   tttcaaagga atattaagaa ggagtatgcc aaaatggtcc aggtcttaca tgcatactgt 600
   atcatttcag caggcatccg tgtaagttgc accaatcagc ttggacaagg aaaacgacag 660
   cctgtggtat gcacaggtgg aagccccagc ataaaggaaa atatcggctc tgtgtttggg 720
   cagaagcagt tgcaaagcct cattcctttt gttcagctgc cccctagtga ctccgtgtgt 780
   gaagagtacg gtttgagctg ttcggatgct ctgcataatc ttttttacat ctcaggtttc 840
   atttcacaat gcacgcatgg agttggaagg agttcaacag acagacagtt tttctttatc 900
   aaccggcggc cttgtgaccc agcaaaggtc tgcagactcg tgaatgaggt ctaccacatg 960
   tataatcgac accagtatcc atttgttgtt cttaacattt ctgttgattc agaatgcgtt 1020
   gatatcaatg ttactccaga taaaaggcaa attttgctac aagaggaaaa gcttttgttg 1080
   gcagttttaa agacctcttt gataggaatg tttgatagtg atgtcaacaa gctaaatgtc 1140
   agtcagcagc cactgctgga tgttgaaggt aacttaataa aaatgcatgc agcggatttg 1200
   gaaaagccca tggtagaaaa gcaggatcaa tccccttcat taaggactgg agaagaaaaa 1260
   aaagacgtgt ccatttccag actgcgagag gccttttctc ttcgtcacac aacagagaac 1320
   aagcctcaca gcccaaagac tccagaacca agaaggagcc ctctaggaca gaaaaggggt 1380
   atgctgtctt ctagcacttc aggtgccatc tctgacaaag gcgtcctgag acctcagaaa 1440
   gaggcagtga gttccagtca cggacccagt gaccctacgg acagagcgga ggtggagaag 1500
   gactcggggc acggcagcac ttccgtggat tctgaggggt tcagcatccc agacacgggc 1560
   agtcactgca gcagcgagta tgcggccagc tccccagggg acaggggctc gcaggaacat 1620
   gtggactctc aggagaaagc gcctgaaact gacgactctt tttcagatgt ggactgccat 1680
   tcaaaccagg aagataccgg atgtaaattt cgagttttgc ctcagccaac taatctcgca 1740
   accccaaaca caaagcgttt taaaaaagaa gaaattcttt ccagttctga catttgtcaa 1800
   aagttagtaa atactcagga catgtcagcc tctcaggttg atgtagctgt gaaaattaat 1860
   aagaaagttg tgcccctgga cttttctatg agttctttag ctaaacgaat aaagcagtta 1920
   catcatgaag cacagcaaag tgaaggggaa cagaattaca ggaagtttag ggcaaagatt 1980
   tgtcctggag aaaatcaagc agccgaagat gaactaagaa aagagataag taaaacgatg 2040
   tttgcagaaa tggaaatcat tggtcagttt aacctgggat ttataataac caaactgaat 2100
   gaggatatct tcatagtgga ccagcatgcc acggacgaga agtataactt cgagatgctg 2160
   cagcagcaca ccgtgctcca ggggcagagg ctcatagcac ctcagactct caacttaact 2220
   gctgttaatg aagctgttct gatagaaaat ctggaaatat ttagaaagaa tggctttgat 2280
   tttgttatcg atgaaaatgc tccagtcact gaaagggcta aactgatttc cttgccaact 2340
   agtaaaaact ggaccttcgg accccaggac gtcgatgaac tgatcttcat gctgagcgac 2400
   agccctgggg tcatgtgccg gccttcccga gtcaagcaga tgtttgcctc cagagcctgc 2460
   cggaagtcgg tgatgattgg gactgctctt aacacaagcg agatgaagaa actgatcacc 2520
   cacatggggg agatggacca cccctggaac tgtccccatg gaaggccaac catgagacac 2580
   atcgccaacc tgggtgtcat ttctcagaac tgaccgtagt cactgtatgg aataattggt 2640
   tttatcgcag atttttatgt tttgaaagac agagtcttca ctaacctttt ttgttttaaa 2700
   atgaaacctg ctacttaaaa aaaatacaca tcacacccat ttaaaagtga tcttgagaac 2760
   cttttcaaac c                                                     2771
<210> 9
   <211> 932
   <212> PRT
   <213> Homo sapiens
<400> 9
<210> 10
   <211> 3063
   <212> DNA
   <213> Homo sapiens
<400> 10
   ggcacgagtg gctgcttgcg gctagtggat ggtaattgcc tgcctcgcgc tagcagcaag 60
   ctgctctgtt aaaagcgaaa atgaaacaat tgcctgcggc aacagttcga ctcctttcaa 120
   gttctcagat catcacttcg gtggtcagtg ttgtaaaaga gcttattgaa aactccttgg 180
   atgctggtgc cacaagcgta gatgttaaac tggagaacta tggatttgat aaaattgagg 240
   tgcgagataa cggggagggt atcaaggctg ttgatgcacc tgtaatggca atgaagtact 300
   acacctcaaa aataaatagt catgaagatc ttgaaaattt gacaacttac ggttttcgtg 360
   gagaagcctt ggggtcaatt tgttgtatag ctgaggtttt aattacaaca agaacggctg 420
   ctgataattt tagcacccag tatgttttag atggcagtgg ccacatactt tctcagaaac 480
   cttcacatct tggtcaaggt acaactgtaa ctgctttaag attatttaag aatctacctg 540
   taagaaagca gttttactca actgcaaaaa aatgtaaaga tgaaataaaa aagatccaag 600
   atctcctcat gagctttggt atccttaaac ctgacttaag gattgtcttt gtacataaca 660
   aggcagttat ttggcagaaa agcagagtat cagatcacaa gatggctctc atgtcagttc 720
   tggggactgc tgttatgaac aatatggaat cctttcagta ccactctgaa gaatctcaga 780
   tttatctcag tggatttctt ccaaagtgtg atgcagacca ctctttcact agtctttcaa 840
   caccagaaag aagtttcatc ttcataaaca gtcgaccagt acatcaaaaa gatatcttaa 900
   agttaatccg acatcattac aatctgaaat gcctaaagga atctactcgt ttgtatcctg 960
   ttttctttct gaaaatcgat gttcctacag ctgatgttga tgtaaattta acaccagata 1020
   aaagccaagt attattacaa aataaggaat ctgttttaat tgctcttgaa aatctgatga 1080
   cgacttgtta tggaccatta cctagtacaa attcttatga aaataataaa acagatgttt 1140
   ccgcagctga catcgttctt agtaaaacag cagaaacaga tgtgcttttt aataaagtgg 1200
   aatcatctgg aaagaattat tcaaatgttg atacttcagt cattccattc caaaatgata 1260
   tgcataatga tgaatctgga aaaaacactg atgattgttt aaatcaccag ataagtattg 1320
   gtgactttgg ttatggtcat tgtagtagtg aaatttctaa cattgataaa aacactaaga 1380
   atgcatttca ggacatttca atgagtaatg tatcatggga gaactctcag acggaatata 1440
   gtaaaacttg ttttataagt tccgttaagc acacccagtc agaaaatggc aataaagacc 1500
   atatagatga gagtggggaa aatgaggaag aagcaggtct tgaaaactct tcggaaattt 1560
   ctgcagatga gtggagcagg ggaaatatac ttaaaaattc agtgggagag aatattgaac 1620
   ctgtgaaaat tttagtgcct gaaaaaagtt taccatgtaa agtaagtaat aataattatc 1680
   caatccctga acaaatgaat cttaatgaag attcatgtaa caaaaaatca aatgtaatag 1740
   ataataaatc tggaaaagtt acagcttatg atttacttag caatcgagta atcaagaaac 1800
   ccatgtcagc aagtgctctt tttgttcaag atcatcgtcc tcagtttctc atagaaaatc 1860
   ctaagactag tttagaggat gcaacactac aaattgaaga actgtggaag acattgagtg 1920
   aagaggaaaa actgaaatat gaagagaagg ctactaaaga cttggaacga tacaatagtc 1980
   aaatgaagag agccattgaa caggagtcac aaatgtcact aaaagatggc agaaaaaaga 2040
   taaaacccac cagcgcatgg aatttggccc agaagcacaa gttaaaaacc tcattatcta 2100
   atcaaccaaa acttgatgaa ctccttcagt cccaaattga aaaaagaagg agtcaaaata 2160
   ttaaaatggt acagatcccc ttttctatga aaaacttaaa aataaatttt aagaaacaaa 2220
   acaaagttga cttagaagag aaggatgaac cttgcttgat ccacaatctc aggtttcctg 2280
   atgcatggct aatgacatcc aaaacagagg taatgttatt aaatccatat agagtagaag 2340
   aagccctgct atttaaaaga cttcttgaga atcataaact tcctgcagag ccactggaaa 2400
   agccaattat gttaacagag agtcttttta atggatctca ttatttagac gttttatata 2460
   aaatgacagc agatgaccaa agatacagtg gatcaactta cctgtctgat cctcgtctta 2520
   cagcgaatgg tttcaagata aaattgatac caggagtttc aattactgaa aattacttgg 2580
   aaatagaagg aatggctaat tgtctcccat tctatggagt agcagattta aaagaaattc 2640
   ttaatgctat attaaacaga aatgcaaagg aagtttatga atgtagacct cgcaaagtga 2700
   taagttattt agagggagaa gcagtgcgtc tatccagaca attacccatg tacttatcaa 2760
   aagaggacat ccaagacatt atctacagaa tgaagcacca gtttggaaat gaaattaaag 2820
   agtgtgttca tggtcgccca ttttttcatc atttaaccta tcttccagaa actacatgat 2880
   taaatatgtt taagaagatt agttaccatt gaaattggtt ctgtcataaa acagcatgag 2940
   tctggtttta aattatcttt gtattatgtg tcacatggtt atttttaaaa tgaggattca 3000
   ctgacttgtt tttatattga aaaaagttcc acgtattgta gaaaacgtaa ataaactaat 3060
   aac                                                               3063
<210> 11
   <211> 934
   <212> PRT
   <213> Homo sapiens
<400> 11
<210> 12
   <211> 3145
   <212> DNA
   <213> Homo sapiens
<400> 12
   ggcgggaaac agcttagtgg gtgtggggtc gcgcattttc ttcaaccagg aggtgaggag 60
   gtttcgacat ggcggtgcag ccgaaggaga cgctgcagtt ggagagcgcg gccgaggtcg 120
   gcttcgtgcg cttctttcag ggcatgccgg agaagccgac caccacagtg cgccttttcg 180
   accggggcga cttctatacg gcgcacggcg aggacgcgct gctggccgcc cgggaggtgt 240
   tcaagaccca gggggtgatc aagtacatgg ggccggcagg agcaaagaat ctgcagagtg 300
   ttgtgcttag taaaatgaat tttgaatctt ttgtaaaaga tcttcttctg gttcgtcagt 360
   atagagttga agtttataag aatagagctg gaaataaggc atccaaggag aatgattggt 420
   atttggcata taaggcttct cctggcaatc tctctcagtt tgaagacatt ctctttggta 480
   acaatgatat gtcagcttcc attggtgttg tgggtgttaa aatgtccgca gttgatggcc 540
   agagacaggt tggagttggg tatgtggatt ccatacagag gaaactagga ctgtgtgaat 600
   tccctgataa tgatcagttc tccaatcttg aggctctcct catccagatt ggaccaaagg 660
   aatgtgtttt acccggagga gagactgctg gagacatggg gaaactgaga cagataattc 720
   aaagaggagg aattctgatc acagaaagaa aaaaagctga cttttccaca aaagacattt 780
   atcaggacct caaccggttg ttgaaaggca aaaagggaga gcagatgaat agtgctgtat 840
   tgccagaaat ggagaatcag gttgcagttt catcactgtc tgcggtaatc aagtttttag 900
   aactcttatc agatgattcc aactttggac agtttgaact gactactttt gacttcagcc 960
   agtatatgaa attggatatt gcagcagtca gagcccttaa cctttttcag ggttctgttg 1020
   aagataccac tggctctcag tctctggctg ccttgctgaa taagtgtaaa acccctcaag 1080
   gacaaagact tgttaaccag tggattaagc agcctctcat ggataagaac agaatagagg 1140
   agagattgaa tttagtggaa gcttttgtag aagatgcaga attgaggcag actttacaag 1200
   aagatttact tcgtcgattc ccagatctta accgacttgc caagaagttt caaagacaag 1260
   cagcaaactt acaagattgt taccgactct atcagggtat aaatcaacta cctaatgtta 1320
   tacaggctct ggaaaaacat gaaggaaaac accagaaatt attgttggca gtttttgtga 1380
   ctcctcttac tgatcttcgt tctgacttct ccaagtttca ggaaatgata gaaacaactt 1440
   tagatatgga tcaggtggaa aaccatgaat tccttgtaaa accttcattt gatcctaatc 1500
   tcagtgaatt aagagaaata atgaatgact tggaaaagaa gatgcagtca acattaataa 1560
   gtgcagccag agatcttggc ttggaccctg gcaaacagat taaactggat tccagtgcac 1620
   agtttggata ttactttcgt gtaacctgta aggaagaaaa agtccttcgt aacaataaaa 1680
   actttagtac tgtagatatc cagaagaatg gtgttaaatt taccaacagc aaattgactt 1740
   ctttaaatga agagtatacc aaaaataaaa cagaatatga agaagcccag gatgccattg 1800
   ttaaagaaat tgtcaatatt tcttcaggct atgtagaacc aatgcagaca ctcaatgatg 1860
   tgttagctca gctagatgct gttgtcagct ttgctcacgt gtcaaatgga gcacctgttc 1920
   catatgtacg accagccatt ttggagaaag gacaaggaag aattatatta aaagcatcca 1980
   ggcatgcttg tgttgaagtt caagatgaaa ttgcatttat tcctaatgac gtatactttg 2040
   aaaaagataa acagatgttc cacatcatta ctggccccaa tatgggaggt aaatcaacat 2100
   atattcgaca aactggggtg atagtactca tggcccaaat tgggtgtttt gtgccatgtg 2160
   agtcagcaga agtgtccatt gtggactgca tcttagcccg agtaggggct ggtgacagtc 2220
   aattgaaagg agtctccacg ttcatggctg aaatgttgga aactgcttct atcctcaggt 2280
   ctgcaaccaa agattcatta ataatcatag atgaattggg aagaggaact tctacctacg 2340
   atggatttgg gttagcatgg gctatatcag aatacattgc aacaaagatt ggtgcttttt 2400
   gcatgtttgc aacccatttt catgaactta ctgccttggc caatcagata ccaactgtta 2460
   ataatctaca tgtcacagca ctcaccactg aagagacctt aactatgctt tatcaggtga 2520
   agaaaggtgt ctgtgatcaa agttttggga ttcatgttgc agagcttgct aatttcccta 2580
   agcatgtaat agagtgtgct aaacagaaag ccctggaact tgaggagttt cagtatattg 2640
   gagaatcgca aggatatgat atcatggaac cagcagcaaa gaagtgctat ctggaaagag 2700
   agcaaggtga aaaaattatt caggagttcc tgtccaaggt gaaacaaatg ccctttactg 2760
   aaatgtcaga agaaaacatc acaataaagt taaaacagct aaaagctgaa gtaatagcaa 2820
   agaataatag ctttgtaaat gaaatcattt cacgaataaa agttactacg tgaaaaatcc 2880
   cagtaatgga atgaaggtaa tattgataag ctattgtctg taatagtttt atattgtttt 2940
   atattaaccc tttttccata gtgttaactg tcagtgccca tgggctatca acttaataag 3000
   atatttagta atattttact ttgaggacat tttcaaagat ttttattttg aaaaatgaga 3060
   gctgtaactg aggactgttt gcaattgaca taggcaataa taagtgatgt gctgaatttt 3120
   ataaataaaa tcatgtagtt tgtgg                                       3145
<210> 13
   <211> 756
   <212> PRT
   <213> Homo sapiens
<400> 13
<210> 14
   <211> 2484
   <212> DNA
   <213> Homo sapiens
<400> 14
   cttggctctt ctggcgccaa aatgtcgttc gtggcagggg ttattcggcg gctggacgag 60
   acagtggtga accgcatcgc ggcgggggaa gttatccagc ggccagctaa tgctatcaaa 120
   gagatgattg agaactgttt agatgcaaaa tccacaagta ttcaagtgat tgttaaagag 180
   ggaggcctga agttgattca gatccaagac aatggcaccg ggatcaggaa agaagatctg 240
   gatattgtat gtgaaaggtt cactactagt aaactgcagt cctttgagga tttagccagt 300
   atttctacct atggctttcg aggtgaggct ttggccagca taagccatgt ggctcatgtt 360
   actattacaa cgaaaacagc tgatggaaag tgtgcataca gagcaagtta ctcagatgga 420
   aaactgaaag cccctcctaa accatgtgct ggcaatcaag ggacccagat cacggtggag 480
   gacctttttt acaacatagc cacgaggaga aaagctttaa aaaatccaag tgaagaatat 540
   gggaaaattt tggaagttgt tggcaggtat tcagtacaca atgcaggcat tagtttctca 600
   gttaaaaaac aaggagagac agtagctgat gttaggacac tacccaatgc ctcaaccgtg 660
   gacaatattc gctccatctt tggaaatgct gttagtcgag aactgataga aattggatgt 720
   gaggataaaa ccctagcctt caaaatgaat ggttacatat ccaatgcaaa ctactcagtg 780
   aagaagtgca tcttcttact cttcatcaac catcgtctgg tagaatcaac ttccttgaga 840
   aaagccatag aaacagtgta tgcagcctat ttgcccaaaa acacacaccc attcctgtac 900
   ctcagtttag aaatcagtcc ccagaatgtg gatgttaatg tgcaccccac aaagcatgaa 960
   gttcacttcc tgcacgagga gagcatcctg gagcgggtgc agcagcacat cgagagcaag 1020
   ctcctgggct ccaattcctc caggatgtac ttcacccaga ctttgctacc aggacttgct 1080
   ggcccctctg gggagatggt taaatccaca acaagtctga cctcgtcttc tacttctgga 1140
   agtagtgata aggtctatgc ccaccagatg gttcgtacag attcccggga acagaagctt 1200
   gatgcatttc tgcagcctct gagcaaaccc ctgtccagtc agccccaggc cattgtcaca 1260
   gaggataaga cagatatttc tagtggcagg gctaggcagc aagatgagga gatgcttgaa 1320
   ctcccagccc ctgctgaagt ggctgccaaa aatcagagct tggaggggga tacaacaaag 1380
   gggacttcag aaatgtcaga gaagagagga cctacttcca gcaaccccag aaagagacat 1440
   cgggaagatt ctgatgtgga aatggtggaa gatgattccc gaaaggaaat gactgcagct 1500
   tgtacccccc ggagaaggat cattaacctc actagtgttt tgagtctcca ggaagaaatt 1560
   aatgagcagg gacatgaggt tctccgggag atgttgcata accactcctt cgtgggctgt 1620
   gtgaatcctc agtgggcctt ggcacagcat caaaccaagt tataccttct caacaccacc 1680
   aagcttagtg aagaactgtt ctaccagata ctcatttatg attttgccaa ttttggtgtt 1740
   ctcaggttat cggagccagc accgctcttt gaccttgcca tgcttgcctt agatagtcca 1800
   gagagtggct ggacagagga agatggtccc aaagaaggac ttgctgaata cattgttgag 1860
   tttctgaaga agaaggctga gatgcttgca gactatttct ctttggaaat tgatgaggaa 1920
   gggaacctga ttggattacc ccttctgatt gacaactatg tgcccccttt ggagggactg 1980
   cctatcttca ttcttcgact agccactgag gtgaattggg acgaagaaaa ggaatgtttt 2040
   gaaagcctca gtaaagaatg cgctatgttc tattccatcc ggaagcagta catatctgag 2100
   gagtcgaccc tctcaggcca gcagagtgaa gtgcctggct ccattccaaa ctcctggaag 2160
   tggactgtgg aacacattgt ctataaagcc ttgcgctcac acattctgcc tcctaaacat 2220
   ttcacagaag atggaaatat cctgcagctt gctaacctgc ctgatctata caaagtcttt 2280
   gagaggtgtt aaatatggtt atttatgcac tgtgggatgt gttcttcttt ctctgtattc 2340
   cgatacaaag tgttgtatca aagtgtgata tacaaagtgt accaacataa gtgttggtag 2400
   cacttaagac ttatacttgc cttctgatag tattccttta tacacagtgg attgattata 2460
   aataaataga tgtgtcttaa cata 2484
<210> 15
   <211> 133
   <212> PRT
   <213> Homo sapiens
<400> 15
   Met Lys Gln Leu Pro Ala Ala Thr Val Arg Leu Leu Ser Ser Ser Gln
   1 5 10 15
   Ile Ile Thr Ser Val Val Ser Val Val Lys Glu Leu Ile Glu Asn Ser
   20 25 30
   Leu Asp Ala Gly Ala Thr Ser Val Asp Val Lys Leu Glu Asn Tyr Gly
   35 40 45
   Phe Asp Lys Ile Glu Val Arg Asp Asn Gly Glu Gly Ile Lys Ala Val
   50 55 60
   Asp Ala Pro Val Met Ala Met Lys Tyr Tyr Thr Ser Lys Ile Asn Ser
   65 70 75 80
   His Glu Asp Leu Glu Asn Leu Thr Thr Tyr Gly Phe Arg Gly Glu Ala
   85 90 95
   Leu Gly Ser Ile Cys Cys Ile Ala Glu Val Leu Ile Thr Thr Arg Thr
   100 105 110
   Ala Ala Asp Asn Phe Ser Thr Gln Tyr Val Leu Asp Gly Ser Gly His
   115 120 125
   Ile Leu Ser Gln Lys
   130
<210> 16
   <211> 426
   <212> DNA
   <213> Homo sapiens
<400> 16
   cgaggcggat cgggtgttgc atccatggag cgagctgaga gctcgagtac agaacctgct 60
   aaggccatca aacctattga tcggaagtca gtccatcaga tttgctctgg gcaggtggta 120
   ctgagtctaa gcactgcggt aaaggagtta gtagaaaaca gtctggatgc tggtgccact 180
   aatattgatc taaagcttaa ggactatgga gtggatctta ttgaagtttc agacaatgga 240
   tgtggggtag aagaagaaaa cttcgaaggc ttaactctga aacatcacac atctaagatt 300
   caagagtttg ccgacctaac tcaggttgaa acttttggct ttcgggggga agctctgagc 360
   tcactttgtg cactgagcga tgtcaccatt tctacctgcc acgcatcggc gaaggttgga 420
   acttga 426
<210> 17
   <211> 181
   <212> DNA
   <213> Artificial Sequence
<220>
   <223> Description of Artificial Sequence:expression cassette
<400> 17
   aagcttccat gtacaggatg caactcctgt cttgcattgc actaagtctt gcacttgtca 60
   caaacagtgc acaaaagctg gagctccacc gcggtggcgg ccgctctaga actagtggat 120
   cccccggggc tgcaggaatt cgatatcaag ccaccatcac catcaccact agtagaagct 180
   t 181

### SEQUENCE LISTING

<110> Nicolaides, Nicholas C.
   Grasso, Luigi
   Sass, Philip M. M.
<120> METHODS FOR GENERATING GENETICALLY ALTERED ANTIGENS
<130> MOR-0016
<140> 00/000,000
   <141> 2000-11-14
<160> 17
<170> Patent In Ver. 2.1
<210> 1
   <211> 86
   <212> DNA
   <213> Artificial Sequence
<220>
   <223> Description of Artificial Sequence: sense primer
<400> 1
   aagcttccat gtacaggatg caactcctgt cttgcattgc actaagtctt gcacttgtca     60
   caaacagtgc acaaaagctg gagctc 86
<210> 2
   <211> 51
   <212> DNA
   <213> Artificial Sequence
<220>
   <223> Description of Artificial Sequence: antisense primer
<400> 2
   ccggatccct actagtggtg atggtgatgg tggcttgata tcgaattcct g 51
<210> 3
   <211> 22
   <212> DNA
   <213> Artificial Sequence
<220>
   <223> Description of Artificial Sequence: sense primer
<400> 3
   catgtacagg atgcaactcc tg 22
<210> 4
   <211> 23
   <212> DNA
   <213> Artificial Sequence
<220>
   <223> Description of Artificial Sequence: antisense primer
<400> 4
   tactagtggt gatggtgatg gtg 23
<210> 5
   <211> 859
   <212> PRT
   <213> Mus musculus
<400> 5
   Met Glu Gln Thr Glu Gly Val Ser Thr Glu Cys Ala Lys Ala Ile Lys
   1 5 10 15
   Pro Ile Asp Gly Lys Ser Val His Gln Ile Cys Ser Gly Gln Val Ile
   20 25 30
   Leu Ser Leu Ser Thr Ala Val Lys Glu Leu Ile Glu Asn Ser Val Asp
   35 40 45
   Ala Gly Ala Thr Thr Ile Asp Leu Arg Leu Lys Asp Tyr Gly Val Asp
   50 55 60
   Leu Ile Glu Val Ser Asp Asn Gly Cys Gly Val Glu Glu Glu Asn Phe
   65 70 75 80
   Glu Gly Leu Ala Leu Lys His His Thr Ser Lys Ile Gln Glu Phe Ala
   85 90 95
   Asp Leu Thr Gln Val Glu Thr Phe Gly Phe Arg Gly Glu Ala Leu Ser
   100 105 110
   Ser Leu Cys Ala Leu Ser Asp Val Thr Ile Ser Thr Cys His Gly Ser
   115 120 125
   Ala Ser Val Gly Thr Arg Leu Val Phe Asp His Asn Gly Lys Ile Thr
   130 135 140
   Gln Lys Thr Pro Tyr Pro Arg Pro Lys Gly Thr Thr Val Ser Val Gln
   145 150 155 160
   His Leu Phe Tyr Thr Leu Pro Val Arg Tyr Lys Glu Phe Gln Arg Asn
   165 170 175
   Ile Lys Lys Glu Tyr Ser Lys Met Val Gln Val Leu Gln Ala Tyr Cys
   180 185 190
   Ile Ile Ser Ala Gly Val Arg Val Ser Cys Thr Asn Gln Leu Gly Gln
   195 200 205
   Gly Lys Arg His Ala Val Val Cys Thr Ser Gly Thr Ser Gly Met Lys
   210 215 220
   Glu Asn Ile Gly Ser Val Phe Gly Gln Lys Gln Leu Gln Ser Leu Ile
   225 230 235 240
   Pro Phe Val Gln Leu Pro Pro Ser Asp Ala Val Cys Glu Glu Tyr Gly
   245 250 255
   Leu Ser Thr Ser Gly Arg His Lys Thr Phe Ser Thr Phe Arg Ala Ser
   260 265 270
   Phe His Ser Ala Arg Thr Ala Pro Gly Gly Val Gln Gln Thr Gly Ser
   275 280 285
   Phe Ser Ser Ser Ile Arg Gly Pro Val Thr Gln Gln Arg Ser Leu Ser
   290 295 300
   Leu Ser Met Arg Phe Tyr His Met Tyr Asn Arg His Gln Tyr Pro Phe
   305 310 315 320
   Val Val Leu Asn Val Ser Val Asp Ser Glu Cys Val Asp Ile Asn Val
   325 330 335
   Thr Pro Asp Lys Arg Gln Ile Leu Leu Gln Glu Glu Lys Leu Leu Leu
   340 345 350
   Ala Val Leu Lys Thr Ser Leu Ile Gly Met Phe Asp Ser Asp Ala Asn
   355 360 365
   Lys Leu Asn Val Asn Gln Gln Pro Leu Leu Asp Val Glu Gly Asn Leu
   370 375 380
   Val Lys Leu His Thr Ala Glu Leu Glu Lys Pro Val Pro Gly Lys Gln
   385 390 395 400
   Asp Asn Ser Pro Ser Leu Lys Ser Thr Ala Asp Glu Lys Arg Val Ala
   405 410 415
   Ser Ile Ser Arg Leu Arg Glu Ala Phe Ser Leu His Pro Thr Lys Glu
   420 425 430
   Ile Lys Ser Arg Gly Pro Glu Thr Ala Glu Leu Thr Arg Ser Phe Pro
   435 440 445
   Ser Glu Lys Arg Gly Val Leu Ser Ser Tyr Pro Ser Asp Val Ile Ser
   450 455 460
   Tyr Arg Gly Leu Arg Gly Ser Gln Asp Lys Leu Val Ser Pro Thr Asp
   465 470 475 480
   Ser Pro Gly Asp Cys Met Asp Arg Glu Lys Ile Glu Lys Asp Ser Gly
   485 490 495
   Leu Ser Ser Thr Ser Ala Gly Ser Glu Glu Glu Phe Ser Thr Pro Glu
   500 505 510
   Val Ala Ser Ser Phe Ser Ser Asp Tyr Asn Val Ser Ser Leu Glu Asp
   515 520 525
   Arg Pro Ser Gln Glu Thr Ile Asn Cys Gly Asp Leu Asp Cys Arg Pro
   530 535 540
   Pro Gly Thr Gly Gln Ser Leu Lys Pro Glu Asp His Gly Tyr Gln Cys
   545 550 555 560
   Lys Ala Leu Pro Leu Ala Arg Leu Ser Pro Thr Asn Ala Lys Arg Phe
   565 570 575
   Lys Thr Glu Glu Arg Pro Ser Asn Val Asn Ile Ser Gln Arg Leu Pro
   580 585 590
   Gly Pro Gln Ser Thr Ser Ala Ala Glu Val Asp Val Ala Ile Lys Met
   595 600 605
   Asn Lys Arg Ile Val Leu Leu Glu Phe Ser Leu Ser Ser Leu Ala Lys
   610 615 620
   Arg Met Lys Gln Leu Gln His Leu Lys Ala Gln Asn Lys His Glu Leu
   625 630 635 640
   Ser Tyr Arg Lys Phe Arg Ala Lys Ile Cys Pro Gly Glu Asn Gln Ala
   645 650 655
   Ala Glu Asp Glu Leu Arg Lys Glu Ile Ser Lys Ser Met Phe Ala Glu
   660 665 670
   Met Glu Ile Leu Gly Gln Phe Asn Leu Gly Phe Ile Val Thr Lys Leu
   675 680 685
   Lys Glu Asp Leu Phe Leu Val Asp Gln His Ala Ala Asp Glu Lys Tyr
   690 695 700
   Asn Phe Glu Met Leu Gln Gln His Thr Val Leu Gln Ala Gln Arg Leu
   705 710 715 720
   Ile Thr Pro Gln Thr Leu Asn Leu Thr Ala Val Asn Glu Ala Val Leu
   725 730 735
   Ile Glu Asn Leu Glu Ile Phe Arg Lys Asn Gly Phe Asp Phe Val Ile
   740 745 750
   Asp Glu Asp Ala Pro Val Thr Glu Arg Ala Lys Leu Ile Ser Leu Pro
   755 760 765
   Thr Ser Lys Asn Trp Thr Phe Gly Pro Gln Asp Ile Asp Glu Leu Ile
   770 775 780
   Phe Met Leu Ser Asp Ser Pro Gly Val Met Cys Arg Pro Ser Arg Val
   785 790 795 800
   Arg Gln Met Phe Ala Ser Arg Ala Cys Arg Lys Ser Val Met Ile Gly
   805 810 815
   Thr Ala Leu Asn Ala Ser Glu Met Lys Lys Leu Ile Thr His Met Gly
   820 825 830
   Glu Met Asp His Pro Trp Asn Cys Pro His Gly Arg Pro Thr Met Arg
   835 840 845
   His Val Ala Asn Leu Asp Val Ile Ser Gln Asn
   850 855
<210> 6
   <211> 3056
   <212> DNA
   <213> Mus musculus
<400> 6
<210> 7
   <211> 862
   <212> PRT
   <213> Homo sapiens
<400> 7
   Met Glu Arg Ala Glu Ser Ser Ser Thr Glu Pro Ala Lys Ala Ile Lys
   1 5 10 15
   Pro Ile Asp Arg Lys Ser Val His Gln Ile Cys Ser Gly Gln Val Val
   20 25 30
   Leu Ser Leu Ser Thr Ala Val Lys Glu Leu Val Glu Asn Ser Leu Asp
   35 40 45
   Ala Gly Ala Thr Asn Ile Asp Leu Lys Leu Lys Asp Tyr Gly Val Asp
   50 55 60
   Leu Ile Glu Val Ser Asp Asn Gly Cys Gly Val Glu Glu Glu Asn Phe
   65 70 75 80
   Glu Gly Leu Thr Leu Lys His His Thr Ser Lys Ile Gln Glu Phe Ala
   85 90 95
   Asp Leu Thr Gln Val Glu Thr Phe Gly Phe Arg Gly Glu Ala Leu Ser
   100 105 110
   Ser Leu Cys Ala Leu Ser Asp Val Thr Ile Ser Thr Cys His Ala Ser
   115 120 125
   Ala Lys Val Gly Thr Arg Leu Met Phe Asp His Asn Gly Lys Ile Ile
   130 135 140
   Gln Lys Thr Pro Tyr Pro Arg Pro Arg Gly Thr Thr Val Ser Val Gln
   145 150 155 160
   Gln Leu Phe Ser Thr Leu Pro Val Arg His Lys Glu Phe Gln Arg Asn
   165 170 175
   Ile Lys Lys Glu Tyr Ala Lys Met Val Gln Val Leu His Ala Tyr Cys
   180 185 190
   Ile Ile Ser Ala Gly Ile Arg Val Ser Cys Thr Asn Gln Leu Gly Gln
   195 200 205
   Gly Lys Arg Gln Pro Val Val Cys Thr Gly Gly Ser Pro Ser Ile Lys
   210 215 220
   Glu Asn Ile Gly Ser Val Phe Gly Gln Lys Gln Leu Gln Ser Leu Ile
   225 230 235 240
   Pro Phe Val Gln Leu Pro Pro Ser Asp Ser Val Cys Glu Glu Tyr Gly
   245 250 255
   Leu Ser Cys Ser Asp Ala Leu His Asn Leu Phe Tyr Ile Ser Gly Phe
   260 265 270
   Ile Ser Gln Cys Thr His Gly Val Gly Arg Ser Ser Thr Asp Arg Gln
   275 280 285
   Phe Phe Phe Ile Asn Arg Arg Pro Cys Asp Pro Ala Lys Val Cys Arg
   290 295 300
   Leu Val Asn Glu Val Tyr His Met Tyr Asn Arg His Gln Tyr Pro Phe
   305 310 315 320
   Val Val Leu Asn Ile Ser Val Asp Ser Glu Cys Val Asp Ile Asn Val
   325 330 335
   Thr Pro Asp Lys Arg Gln Ile Leu Leu Gln Glu Glu Lys Leu Leu Leu
   340 345 350
   Ala Val Leu Lys Thr Ser Leu Ile Gly Met Phe Asp Ser Asp Val Asn
   355 360 365
   Lys Leu Asn Val Ser Gln Gln Pro Leu Leu Asp Val Glu Gly Asn Leu
   370 375 380
   Ile Lys Met His Ala Ala Asp Leu Glu Lys Pro Met Val Glu Lys Gln
   385 390 395 400
   Asp Gln Ser Pro Ser Leu Arg Thr Gly Glu Glu Lys Lys Asp Val Ser
   405 410 415
   Ile Ser Arg Leu Arg Glu Ala Phe Ser Leu Arg His Thr Thr Glu Asn
   420 425 430
   Lys Pro His Ser Pro Lys Thr Pro Glu Pro Arg Arg Ser Pro Leu Gly
   435 440 445
   Gln Lys Arg Gly Met Leu Ser Ser Ser Thr Ser Gly Ala Ile Ser Asp
   450 455 460
   Lys Gly Val Leu Arg Pro Gln Lys Glu Ala Val Ser Ser Ser His Gly
   465 470 475 480
   Pro Ser Asp Pro Thr Asp Arg Ala Glu Val Glu Lys Asp Ser Gly His
   485 490 495
   Gly Ser Thr Ser Val Asp Ser Glu Gly Phe Ser Ile Pro Asp Thr Gly
   500 505 510
   Ser His Cys Ser Ser Glu Tyr Ala Ala Ser Ser Pro Gly Asp Arg Gly
   515 520 525
   Ser Gln Glu His Val Asp Ser Gln Glu Lys Ala Pro Glu Thr Asp Asp
   530 535 540
   Ser Phe Ser Asp Val Asp Cys His Ser Asn Gln Glu Asp Thr Gly Cys
   545 550 555 560
   Lys Phe Arg Val Leu Pro Gln Pro Thr Asn Leu Ala Thr Pro Asn Thr
   565 570 575
   Lys Arg Phe Lys Lys Glu Glu Ile Leu Ser Ser Ser Asp Ile Cys Gln
   580 585 590
   Lys Leu Val Asn Thr Gln Asp Met Ser Ala Ser Gln Val Asp Val Ala
   595 600 605
   Val Lys Ile Asn Lys Lys Val Val Pro Leu Asp Phe Ser Met Ser Ser
   610 615 620
   Leu Ala Lys Arg Ile Lys Gln Leu His His Glu Ala Gln Gln Ser Glu
   625 630 635 640
   Gly Glu Gln Asn Tyr Arg Lys Phe Arg Ala Lys Ile Cys Pro Gly Glu
   645 650 655
   Asn Gln Ala Ala Glu Asp Glu Leu Arg Lys Glu Ile Ser Lys Thr Met
   660 665 670
   Phe Ala Glu Met Glu Ile Ile Gly Gln Phe Asn Leu Gly Phe Ile Ile
   675 680 685
   Thr Lys Leu Asn Glu Asp Ile Phe Ile Val Asp Gln His Ala Thr Asp
   690 695 700
   Glu Lys Tyr Asn Phe Glu Met Leu Gln Gln His Thr Val Leu Gln Gly
   705 710 715 720
   Gln Arg Leu Ile Ala Pro Gln Thr Leu Asn Leu Thr Ala Val Asn Glu
   725 730 735
   Ala Val Leu Ile Glu Asn Leu Glu Ile Phe Arg Lys Asn Gly Phe Asp
   740 745 750
   Phe Val Ile Asp Glu Asn Ala Pro Val Thr Glu Arg Ala Lys Leu Ile
   755 760 765
   Ser Leu Pro Thr Ser Lys Asn Trp Thr Phe Gly Pro Gln Asp Val Asp
   770 775 780
   Glu Leu Ile Phe Met Leu Ser Asp Ser Pro Gly Val Met Cys Arg Pro
   785 790 795 800
   Ser Arg Val Lys Gln Met Phe Ala Ser Arg Ala Cys Arg Lys Ser Val
   805 810 815
   Met Ile Gly Thr Ala Leu Asn Thr Ser Glu Met Lys Lys Leu Ile Thr
   820 825 830
   His Met Gly Glu Met Asp His Pro Trp Asn Cys Pro His Gly Arg Pro
   835 840 845
   Thr Met Arg His Ile Ala Asn Leu Gly Val Ile Ser Gln Asn
   850 855 860
<210> 8
   <211> 2771
   <212> DNA
   <213> Homo sapiens
<400> 8
<210> 9
   <211> 932
   <212> PRT
   <213> Homo sapiens
<400> 9
<210> 10
   <211> 3063
   <212> DNA
   <213> Homo sapiens
<400> 10
<210> 11
   <211> 934
   <212> PRT
   <213> Homo sapiens
<400> 11
<210> 12
   <211> 3145
   <212> DNA
   <213> Homo sapiens
<400> 12
<210> 13
   <211> 756
   <212> PRT
   <213> Homo sapiens
<400> 13
<210> 14
   <211> 2484
   <212> DNA
   <213> Homo sapiens
<400> 14
<210> 15
   <211> 133
   <212> PRT
   <213> Homo sapiens
<400> 15
   Met Glu Arg Ala Glu Ser Ser Ser Thr Glu Pro Ala Lys Ala Ile Lys
   1 5 10 15
   Pro Ile Asp Arg Lys Ser Val His Gln Ile Cys Ser Gly Gln Val Val
   20 25 30
   Leu Ser Leu Ser Thr Ala Val Lys Glu Leu Val Glu Asn Ser Leu Asp
   35 40 45
   Ala Gly Ala Thr Asn Ile Asp Leu Lys Leu Lys Asp Tyr Gly Val Asp
   50 55 60
   Leu Ile Glu Val Ser Asp Asn Gly Cys Gly Val Glu Glu Glu Asn Phe
   65 70 75 80
   Glu Gly Leu Thr Leu Lys His His Thr Ser Lys Ile Gln Glu Phe Ala
   85 90 95
   Asp Leu Thr Gln Val Glu Thr Phe Gly Phe Arg Gly Glu Ala Leu Ser
   100 105 110
   Ser Leu Cys Ala Leu Ser Asp Val Thr Ile Ser Thr Cys His Ala Ser
   115 120 125
   Ala Lys Val Gly Thr
   130
<210> 16
   <211> 426
   <212> DNA
   <213> Homo sapiens
<400> 16
<210> 17
   <211> 181
   <212> DNA
   <213> Artificial Sequence
<220>
   <223> Description of Artificial Sequence: expression cassette
<400> 17

### SEQUENCE LISTING

<110> Nicolaides, Nicholas C.
   Grasso, Luigi
   Sass, Philip M. M.
<120> METHODS FOR GENERATING GENETICALLY ALTERED ANTIGENS
<130> MOR-0016
<140> 00/000,000
   <141> 2000-11-14
<160> 17
<170> PatentIn Ver. 2.1
<210> 1
   <211> 86
   <212> DNA
   <213> Artificial Sequence
<220>
   <223> Description of Artificial Sequence: sense primer
<400> 1
<210> 2
   <211> 51
   <212> DNA
   <213> Artificial Sequence
<220>
   <223> Description of Artificial Sequence: antisense primer
<400> 2
   ccggatccct actagtggtg atggtgatgg tggcttgata tcgaattcct 51
<210> 3
   <211> 22
   <212> DNA
   <213> Artificial Sequence
<220>
   <223> Description of Artificial Sequence: sense primer
<400> 3
   catgtacagg atgcaactcc 22
<210> 4
   <211> 23
   <212> DNA
   <213> Artificial Sequence
<220>
   <223> Description of Artificial Sequence: antisense primer
<400> 4
   tactagtggt gatggtggatg gtg 23
<210> 5
   <211> 859
   <212> PRT
   <213> Mus musculus
<400> 5
   Met Glu Gln Thr Glu Gly Val Ser Thr Glu Cys Ala Lys Ala Ile Lys
   1               5                   10                  15
   Pro Ile Asp Gly Lys Ser Val His Gln Ile Cys Ser Gly Gln Val Ile
               20                  25                  30
   Leu Ser Leu Ser Thr Ala Val Lys Glu Leu Ile Glu Asn Ser Val Asp
           35                  40                  45
   Ala Gly Ala Thr Thr Ile Asp Leu Arg Leu Lys Asp Tyr Gly Val Asp
       50                  55                  60
   Leu Ile Glu Val Ser Asp Asn Gly Cys Gly Val Glu Glu Glu Asn Phe
   65                  70                  75                  80
   Glu Gly Leu Ala Leu Lys His His Thr Ser Lys Ile Gln Glu Phe Ala
   85 90 95
   Asp Leu Thr Gln Val Glu Thr Phe Gly Phe Arg Gly Glu Ala Leu Ser
   100 105 110
   Ser Leu Cys Ala Leu Ser Asp Val Thr Ile Ser Thr Cys His Gly Ser
   115 120 125
   Ala Ser Val Gly Thr Arg Leu Val Phe Asp His Asn Gly Lys Ile Thr
   130 135 140
   Gln Lys Thr Pro Tyr Pro Arg Pro Lys Gly Thr Thr Val Ser Val Gln
   145 150 155 160
   His Leu Phe Tyr Thr Leu Pro Val Arg Tyr Lys Glu Phe Gln Arg Asn
   165 170 175
   Ile Lys Lys Glu Tyr Ser Lys Met Val Gln Val Leu Gln Ala Tyr Cys
   180 185 190
   Ile Ile Ser Ala Gly Val Arg Val Ser Cys Thr Asn Gln Leu Gly Gln
   195 200 205
   Gly Lys Arg His Ala Val Val Cys Thr Ser Gly Thr Ser Gly Met Lys
   210 215 220
   Glu Asn Ile Gly Ser Val Phe Gly Gln Lys Gln Leu Gln Ser Leu Ile
   225 230 235 240
   Pro Phe Val Gln Leu Pro Pro Ser Asp Ala Val Cys Glu Glu Tyr Gly
   245 250 255
   Leu Ser Thr Ser Gly Arg His Lys Thr Phe Ser Thr Phe Arg Ala Ser
   260 265 270
   Phe His Ser Ala Arg Thr Ala Pro Gly Gly Val Gln Gln Thr Gly Ser
   275 280 285
   Phe Ser Ser Ser Ile Arg Gly Pro Val Thr Gln Gln Arg Ser Leu Ser
   290 295 300
   Leu Ser Met Arg Phe Tyr His Met Tyr Asn Arg His Gln Tyr Pro Phe
   305 310 315 320
   Val Val Leu Asn Val Ser Val Asp Ser Glu Cys Val Asp Ile Asn Val
   325 330 335
   Thr Pro Asp Lys Arg Gln Ile Leu Leu Gln Glu Glu Lys Leu Leu Leu
   340 345 350
   Ala Val Leu Lys Thr Ser Leu Ile Gly Met Phe Asp Ser Asp Ala Asn
   355 360 365
   Lys Leu Asn Val Asn Gln Gln Pro Leu Leu Asp Val Glu Gly Asn Leu
   370 375 380
   Val Lys Leu His Thr Ala Glu Leu Glu Lys Pro Val Pro Gly Lys Gln
   385 390 395 400
   Asp Asn Ser Pro Ser Leu Lys Ser Thr Ala Asp Glu Lys Arg Val Ala
   405 410 415
   Ser Ile Ser Arg Leu Arg Glu Ala Phe Ser Leu His Pro Thr Lys Glu
   420 425 430
   Ile Lys Ser Arg Gly Pro Glu Thr Ala Glu Leu Thr Arg Ser Phe Pro
   435 440 445
   Ser Glu Lys Arg Gly Val Leu Ser Ser Tyr Pro Ser Asp Val Ile Ser
   450 455 460
   Tyr Arg Gly Leu Arg Gly Ser Gln Asp Lys Leu Val Ser Pro Thr Asp
   465 470 475 480
   Ser Pro Gly Asp Cys Met Asp Arg Glu Lys Ile Glu Lys Asp Ser Gly
   485 490 495
   Leu Ser Ser Thr Ser Ala Gly Ser Glu Glu Glu Phe Ser Thr Pro Glu
   500 505 510
   Val Ala Ser Ser Phe Ser Ser Asp Tyr Asn Val Ser Ser Leu Glu Asp
   515 520 525
   Arg Pro Ser Gln Glu Thr Ile Asn Cys Gly Asp Leu Asp Cys Arg Pro
   530 535 540
   Pro Gly Thr Gly Gln Ser Leu Lys Pro Glu Asp His Gly Tyr Gln Cys
   545 550 555 560
   Lys Ala Leu Pro Leu Ala Arg Leu Ser Pro Thr Asn Ala Lys Arg Phe
   565 570 575
   Lys Thr Glu Glu Arg Pro Ser Asn Val Asn Ile Ser Gln Arg Leu Pro
   580 585 590
   Gly Pro Gln Ser Thr Ser Ala Ala Glu Val Asp Val Ala Ile Lys Met
   595 600 605
   Asn Lys Arg Ile Val Leu Leu Glu Phe Ser Leu Ser Ser Leu Ala Lys
   610 615 620
   Arg Met Lys Gln Leu Gln His Leu Lys Ala Gln Asn Lys His Glu Leu
   625 630 635 640
   Ser Tyr Arg Lys Phe Arg Ala Lys Ile Cys Pro Gly Glu Asn Gln Ala
   645 650 655
   Ala Glu Asp Glu Leu Arg Lys Glu Ile Ser Lys Ser Met Phe Ala Glu
   660 665 670
   Met Glu Ile Leu Gly Gln Phe Asn Leu Gly Phe Ile Val Thr Lys Leu
   675 680 685
   Lys Glu Asp Leu Phe Leu Val Asp Gln His Ala Ala Asp Glu Lys Tyr
   690 695 700
   Asn Phe Glu Met Leu Gln Gln His Thr Val Leu Gln Ala Gln Arg Leu
   705 710 715 720
   Ile Thr Pro Gln Thr Leu Asn Leu Thr Ala Val Asn Glu Ala Val Leu
   725 730 735
   Ile Glu Asn Leu Glu Ile Phe Arg Lys Asn Gly Phe Asp Phe Val Ile
   740 745 750
   Asp Glu Asp Ala Pro Val Thr Glu Arg Ala Lys Leu Ile Ser Leu Pro
   755 760 765
   Thr Ser Lys Asn Trp Thr Phe Gly Pro Gln Asp Ile Asp Glu Leu Ile
   770 775 780
   Phe Met Leu Ser Asp Ser Pro Gly Val Met Cys Arg Pro Ser Arg Val
   785 790 795 800
   Arg Gln Met Phe Ala Ser Arg Ala Cys Arg Lys Ser Val Met Ile Gly
   805 810 815
   Thr Ala Leu Asn Ala Ser Glu Met Lys Lys Leu Ile Thr His Met Gly
   820 825 830
   Glu Met Asp His Pro Trp Asn Cys Pro His Gly Arg Pro Thr Met Arg
   835 840 845
   His Val Ala Asn Leu Asp Val Ile Ser Gln Asn
   850 855
<210> 6
   <211> 3056
   <212> DNA
   <213> Mus musculus
<400> 6
<210> 7
   <211> 862
   <212> PRT
   <213> Homo sapiens
<400> 7
   Met Glu Arg Ala Glu Ser Ser Ser Thr Glu Pro Ala Lys Ala Ile Lys
   1 5 10 15
   Pro Ile Asp Arg Lys Ser Val His Gln Ile Cys Ser Gly Gln Val Val
   20 25 30
   Leu Ser Leu Ser Thr Ala Val Lys Glu Leu Val Glu Asn Ser Leu Asp
   35 40 45
   Ala Gly Ala Thr Asn Ile Asp Leu Lys Leu Lys Asp Tyr Gly Val Asp
   50 55 60
   Leu Ile Glu Val Ser Asp Asn Gly Cys Gly Val Glu Glu Glu Asn Phe
   65 70 75 80
   Glu Gly Leu Thr Leu Lys His His Thr Ser Lys Ile Gln Glu Phe Ala
   85 90 95
   Asp Leu Thr Gln Val Glu Thr Phe Gly Phe Arg Gly Glu Ala Leu Ser
   100 105 110
   Ser Leu Cys Ala Leu Ser Asp Val Thr Ile Ser Thr Cys His Ala Ser
   115 120 125
   Ala Lys Val Gly Thr Arg Leu Met Phe Asp His Asn Gly Lys Ile Ile
   130 135 140
   Gln Lys Thr Pro Tyr Pro Arg Pro Arg Gly Thr Thr Val Ser Val Gln
   145 150 155 160
   Gln Leu Phe Ser Thr Leu Pro Val Arg His Lys Glu Phe Gln Arg Asn
   165 170 175
   Ile Lys Lys Glu Tyr Ala Lys Met Val Gln Val Leu His Ala Tyr Cys
   180 185 190
   Ile Ile Ser Ala Gly Ile Arg Val Ser Cys Thr Asn Gln Leu Gly Gln
   195 200 205
   Gly Lys Arg Gln Pro Val Val Cys Thr Gly Gly Ser Pro Ser Ile Lys
   210 215 220
   Glu Asn Ile Gly Ser Val Phe Gly Gln Lys Gln Leu Gln Ser Leu Ile
   225 230 235 240
   Pro Phe Val Gln Leu Pro Pro Ser Asp Ser Val Cys Glu Glu Tyr Gly
   245 250 255
   Leu Ser Cys Ser Asp Ala Leu His Asn Leu Phe Tyr Ile Ser Gly Phe
   260 265 270
   Ile Ser Gln Cys Thr His Gly Val Gly Arg Ser Ser Thr Asp Arg Gln
   275 280 285
   Phe Phe Phe Ile Asn Arg Arg Pro Cys Asp Pro Ala Lys Val Cys Arg
   290 295 300
   Leu Val Asn Glu Val Tyr His Met Tyr Asn Arg His Gln Tyr Pro Phe
   305 310 315 320
   Val Val Leu Asn Ile Ser Val Asp Ser Glu Cys Val Asp Ile Asn Val
   325 330 335
   Thr Pro Asp Lys Arg Gln Ile Leu Leu Gln Glu Glu Lys Leu Leu Leu
   340 345 350
   Ala Val Leu Lys Thr Ser Leu Ile Gly Met Phe Asp Ser Asp Val Asn
   355 360 365
   Lys Leu Asn Val Ser Gln Gln Pro Leu Leu Asp Val Glu Gly Asn Leu
   370 375 380
   Ile Lys Met His Ala Ala Asp Leu Glu Lys Pro Met Val Glu Lys Gln
   385 390 395 400
   Asp Gln Ser Pro Ser Leu Arg Thr Gly Glu Glu Lys Lys Asp Val Ser
   405 410 415
   Ile Ser Arg Leu Arg Glu Ala Phe Ser Leu Arg His Thr Thr Glu Asn
   420 425 430
   Lys Pro His Ser Pro Lys Thr Pro Glu Pro Arg Arg Ser Pro Leu Gly
   435 440 445
   Gln Lys Arg Gly Met Leu Ser Ser Ser Thr Ser Gly Ala Ile Ser Asp
   450 455 460
   Lys Gly Val Leu Arg Pro Gln Lys Glu Ala Val Ser Ser Ser His Gly
   465 470 475 480
   Pro Ser Asp Pro Thr Asp Arg Ala Glu Val Glu Lys Asp Ser Gly His
   485 490 495
   Gly Ser Thr Ser Val Asp Ser Glu Gly Phe Ser Ile Pro Asp Thr Gly
   500 505 510
   Ser His Cys Ser Ser Glu Tyr Ala Ala Ser Ser Pro Gly Asp Arg Gly
   515 520 525
   Ser Gln Glu His Val Asp Ser Gln Glu Lys Ala Pro Glu Thr Asp Asp
   530 535 540
   Ser Phe Ser Asp Val Asp Cys His Ser Asn Gln Glu Asp Thr Gly Cys
   545 550 555 560
   Lys Phe Arg Val Leu Pro Gln Pro Thr Asn Leu Ala Thr Pro Asn Thr
   565 570 575
   Lys Arg Phe Lys Lys Glu Glu Ile Leu Ser Ser Ser Asp Ile Cys Gln
   580 585 590
   Lys Leu Val Asn Thr Gln Asp Met Ser Ala Ser Gln Val Asp Val Ala
   595 600 605
   Val Lys Ile Asn Lys Lys Val Val Pro Leu Asp Phe Ser Met Ser Ser
   610 615 620
   Leu Ala Lys Arg Ile Lys Gln Leu His His Glu Ala Gln Gln Ser Glu
   625 630 635 640
   Gly Glu Gln Asn Tyr Arg Lys Phe Arg Ala Lys Ile Cys Pro Gly Glu
   645 650 655
   Asn Gln Ala Ala Glu Asp Glu Leu Arg Lys Glu Ile Ser Lys Thr Met
   660 665 670
   Phe Ala Glu Met Glu Ile Ile Gly Gln Phe Asn Leu Gly Phe Ile Ile
   675 680 685
   Thr Lys Leu Asn Glu Asp Ile Phe Ile Val Asp Gln His Ala Thr Asp
   690 695 700
   Glu Lys Tyr Asn Phe Glu Met Leu Gln Gln His Thr Val Leu Gln Gly
   705 710 715 720
   Gln Arg Leu Ile Ala Pro Gln Thr Leu Asn Leu Thr Ala Val Asn Glu
   725 730 735
   Ala Val Leu Ile Glu Asn Leu Glu Ile Phe Arg Lys Asn Gly Phe Asp
   740 745 750
   Phe Val Ile Asp Glu Asn Ala Pro Val Thr Glu Arg Ala Lys Leu Ile
   755 760 765
   Ser Leu Pro Thr Ser Lys Asn Trp Thr Phe Gly Pro Gln Asp Val Asp
   770 775 780
   Glu Leu Ile Phe Met Leu Ser Asp Ser Pro Gly Val Met Cys Arg Pro
   785 790 795 800
   Ser Arg Val Lys Gln Met Phe Ala Ser Arg Ala Cys Arg Lys Ser Val
   805 810 815
   Met Ile Gly Thr Ala Leu Asn Thr Ser Glu Met Lys Lys Leu Ile Thr
   820 825 830
   His Met Gly Glu Met Asp His Pro Trp Asn Cys Pro His Gly Arg Pro
   835 840 845
   Thr Met Arg His Ile Ala Asn Leu Gly Val Ile Ser Gln Asn
   850 855 860
<210> 8
   <211> 2771
   <212> DNA
   <213> Homo sapiens
<400> 8
<210> 9
   <211> 932
   <212> PRT
   <213> Homo sapiens
<400> 9
<210> 10
   <211> 3063
   <212> DNA
   <213> Homo sapiens
<400> 10
<210> 11
   <211> 934
   <212> PRT
   <213> Homo sapiens
<400> 11
<210> 12
   <211> 3145
   <212> DNA
   <213> Homo sapiens
<400> 12
<210> 13
   <211> 756
   <212> PRT
   <213> Homo sapiens
<400> 13
<210> 14
   <211> 2484
   <212> DNA
   <213> Homo sapiens
<400> 14
<210> 15
   <211> 133
   <212> PRT
   <213> Homo sapiens
<400> 15
   Met Glu Arg Ala Glu Ser Ser Ser Thr Glu Pro Ala Lys Ala Ile Lys
   1 5 10 15
   Pro Ile Asp Arg Lys Ser Val His Gln Ile Cys Ser Gly Gln Val Val
   20 25 30
   Leu Ser Leu Ser Thr Ala Val Lys Glu Leu Val Glu Asn Ser Leu Asp
   35 40 45
   Ala Gly Ala Thr Asn Ile Asp Leu Lys Leu Lys Asp Tyr Gly Val Asp
   50 55 60
   Leu Ile Glu Val Ser Asp Asn Gly Cys Gly Val Glu Glu Glu Asn Phe
   65 70 75 80
   Glu Gly Leu Thr Leu Lys His His Thr Ser Lys Ile Gln Glu Phe Ala
   85 90 95
   Asp Leu Thr Gln Val Glu Thr Phe Gly Phe Arg Gly Glu Ala Leu Ser
   100 105 110
   Ser Leu Cys Ala Leu Ser Asp Val Thr Ile Ser Thr Cys His Ala Ser
   115 120 125
   Ala Lys Val Gly Thr
   130
<210> 16
   <211> 426
   <212> DNA
   <213> Homo sapiens
<400> 16
<210> 17
   <211> 181
   <212> DNA
   <213> Artificial Sequence
<220>
   <223> Description of Artificial Sequence: expression cassette
<400> 17

## Description

### TECHNICAL FIELD OF THE INVENTION

The invention is related to the area of genetic alterations of antigens as potent vaccines. In particular it is related to the field of mutagenesis.

### BACKGROUND OF THE INVENTION

The use of vaccines to build immunity against foreign and/or endogenous polypeptides provides an effective and selective strategy for treating the underlying cause of disease. In particular is the use of killed viruses such as the polio mellitus and the Hepatitis B virus (John T.J. (2000) *New Engl. J. Med.* 14:806-807). Standard methods for generating vaccines against candidate pathogenic organisms or molecules are known by those skilled in the art. Vaccines for human use are developed in animal models to survey for the ability of killed or defective whole agents such as parasites, viruses or recombinant polypeptides to cause immunity against infection of the pathogenic agent (Boyce *T.G. et al.* (2000) *Vaccine* 19:217-226). Briefly, rodents such as mice or rats are injected with a purified antigen in the presence of adjuvant to generate an immune response (Boyce T.G. *et al.* (2000) *Vaccine* 19:217-226). Unfortunately, not all antigens are capable of eliciting a strong immune response when injected into a host organism (Hoshino Y. and A.Z. Kapikian (2000) *J. Health Popul. Nutr.* 18:5-14; Orenstein W.A. *et al.* (2000) *Am. J. Public Health* 90:1521-525; Lechmann M. and T.J. Liang (2000) *Semin. Liver Dis.* 20:211-226). While the reasons for the lack of immune response are not clear, some factors, such as the lack of T-cell epitopes which are important for stimulating cellular-mediated immune responses, may be absent within a given antigen (Ausiello *C.M. et al.* (1999) *Infect. Immun.*67:4064-4071; Brosstoff S. (1995) *Adv. Exp. Med. Biol..* 383:249-254). In the case of parasitic infections, the development of effective vaccines has been hampered by the presence of many different developmental stages that occur within an infected host and that a diverse array of allelic forms occurs within genes encoding for prominent surface antigens (MALARIA OBSTACLES AND OPPORTUNITIES, Oaks, S.C. *et al.,* Eds., National Academy Press, p 1, 1991; Anders, R.F. "Vaccines Against Asexual Blood Stages of *Plasmodium falciparum"* NEW GENERATION VACCINES, 2^{nd} Ed., Anders, R.F., pp. 1035-1055, 1997). It is believed by many skilled in the art that the generation of highly antigenic polypeptides may overcome these limitations and produce a protective immune response to pathogens (McLeod R. *et al.* (1995) *Curr. Opin. Immunol.* 7:539-552).

A method for generating diverse sequences within a polypeptide would be useful for the creation of more potent therapeutic agents. Moreover, the generation of randomly altered nucleotides and encoded polypeptide residues throughout an entire antigen molecule may result in new reagents that are: 1) more antigenic; 2) more immunogenic; and 3) have beneficial pharmacokinetic properties.

CA-A-2240609 disclosed as a method for producing hypermutable cells and organisms by introducing a dominant negative allele of a human mis-match repair gene.

### SUMMARY OF THE INVENTION

The present invention provides a method for making a hypermutated antigen, comprising introducing into an isolated mammalian cell that expresses a preselected antigen a polynucleotide comprising a dominant negative allele of a mismatch repair gene, wherein said hypermutated antigen comprises increased antigenicity or increased immunogenicity relative to said preselected antigen.

The present invention also provides a method for making randomly altered forms of a secreted antigen, comprising introducing a polynucleotide encoding a tagged antigen into a mismatch repair defective cell.

The present invention further provides a method of producing a mutated antigen in a reversibly unstable cell, comprising
introducing into a cell containing a preselected antigen of interest, an inducible expression vector comprising a polynucleotide encoding a dominant negative allele of a mismatch repair gene;
inducing said cell to express said dominant negative mismatch repair gene; and
detecting increased antigenicity or increased immunogenicity of said mutated antigen relative to said preselected antigen.

### BRIEF DESCRIPTION OF THE DRAWINGS

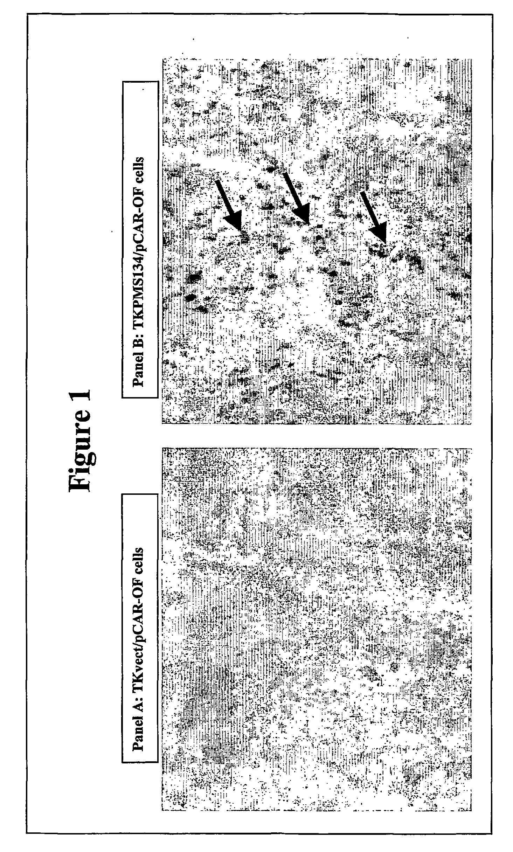

**Figure 1:** *In situ* β-galactosidase staining of TKPMS134/pCAR-OF or TKvect/pCAR-OF cells to assay for MMR defective cells containing genetically altered β-galactosidase genes. Arrows indicate Blue (β-galactosidase positive) cells.

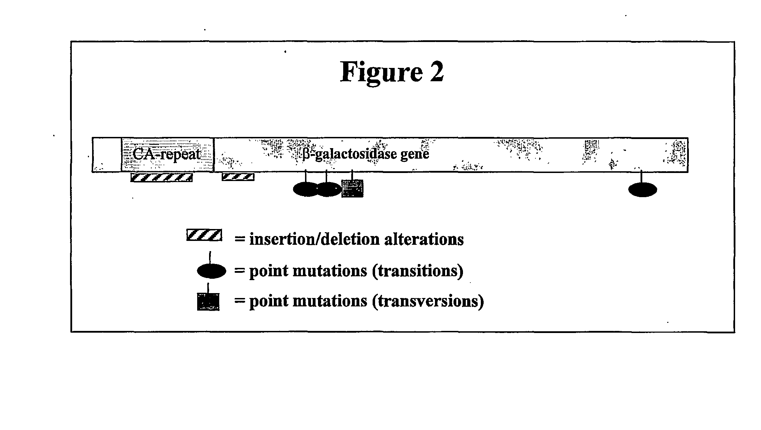

**Figure 2:** Schematic representation of sequence alterations of the□ β-galactosidase gene produced by MMR defective host cells

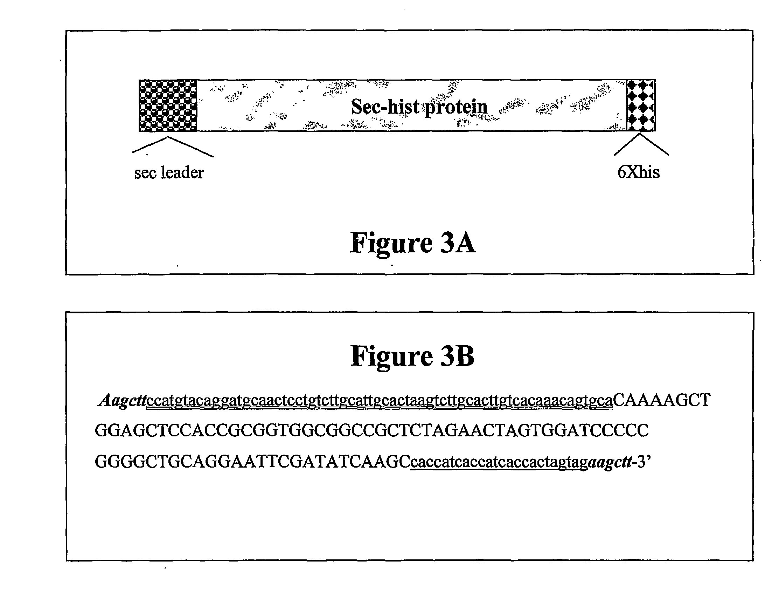

**Figure 3**: Schematic representation of sec-hist secretion proteins for screening of structurally altered antigenic polypeptides and the sec-hist expression cassette (SEQ ID NO:17). Panel A: Schematic representation of sec-hist protein; Panel B: Sequence of sec-hist expression cassette. In Panel B, the italic bold sequence represents a HindIII site for subcloning; the double underlined sequence on the 5' end represents leader sequence from the human IL-2; the underlined sequence on the 3' end represents the poly histidine sequence followed by 2 termination codons; sequence in capital letters represents sequence from the polylinker region of pUC18; the polylinker contains the following restriction enzymes for cloning cDNAs: SacI-SacII-NotI-XbaI-SpeI-BamHI-SmaI-PstI-EcoRI.

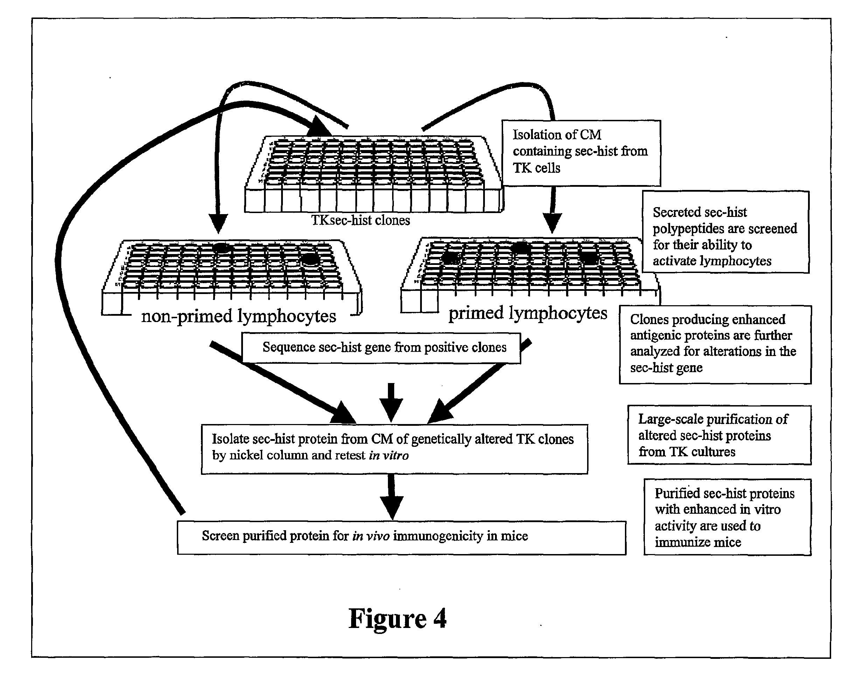

Figure 4: Schematic diagram for high throughput screening of conditioned medium from TK clones for the identification of antigenic sec-hist polypeptides. Assays employ an *in vitro* antigenicity test using splenocytes from naïve mice (non-primed) and *antigen*-exposed (primed) mice. Clones exhibiting positive CM are then genetically analyzed to confirm structural alterations within the sec-hist sequence, followed by protein purification and retesting of purified proteins. Purified proteins with the best stimulatory activity are then screened *in vivo* for immunogenicity. The screening assay can be repeated for several rounds to add additional alterations within the antigen (long arrow).

The inventors have discovered a method for developing hypermutable cells producing therapeutic antigens by taking advantage of the conserved mismatch repair (MMR) process of host cells. Dominant negative alleles of such genes, when introduced into cells or transgenic animals, increase the rate of spontaneous mutations by reducing the effectiveness of DNA repair and thereby render the cells or animals hypermutable. Hypermutable cells or animals can then be utilized to develop new mutations in a gene or genes of interest. Blocking MMR in cells producing antigens (including, but not limited to, isolated mammalian cells, plant cells, yeast cells, and prokaryotic cells) can enhance the rate of mutation within the gene encoding for the antigen that can be screened to identify clones producing structurally altered polypeptides with enhanced antigenicitiy and immunogenicity.

In one aspect of the invention, the methods are useful for the production of antigens that have increased antigenicity and/or immunogenicity. Such antigens may be used as immunogens to elicit immune responses in animals against these antigens.

The antigens may be derived from, for example, pathogenic organisms or cancer cells such that an immune response is directed against the pathogenic organism or cancer cell and exerts an effect on the organism or cancer cell. The effect may be, for example, to prevent, inhibit or terminate the growth of the pathogenic organism or cancer cell when an immunogenic amount of the antigen is administered to an animal.

The pathogenic organisms from which antigens may be derived include bacteria, fungi, parasitic protozoa, helminths, and viruses. Non-limiting examples include species of the following genera: *Staphylococcus, Streptococcus, Bacillus, Bordetella, Clostridium, Escherichia, Haemophilus, Helicobacter, Klebsiella, Listeria, Salmonella, Vibrio, Yersinia, Neisseria, Treponema, Borrelia, Corynebacterium, Mycobacterium, Mycoplasma, Chlamydia, Acremonium, Aspergillus, Blastomyces, Candida, Acanthamoeba, Ascaris, Babesia, Cryptosporidium, Echinococcus, Entamoeba, Giardia, Necator, Ancylostoma, Unicinaria, Leishmania, Onchocerca, Plasmodium, Schistosoma, Strongyloides, Taenia, Toxoplasma, Trichinella, Trichomonas, Trichuris, Trypanosoma, Dirofilaria, Brugia, Wuchereria,* and *Eimeria.* Non-limiting examples of viruses include adenovirus, arborviruses, coronavirus, cytomegalovirus, enteroviruses, Epstein-Barr virus, hepatitis viruses, herpes viruses, immunodeficiency viruses *(e.g. ,* HIV, FIV SIV), papilloma viruses, T-cell leukemia viruses, influenza viruses, mumps viruses, parainfluenzae viruses, parvoviruses, poxviruses, Rabies virus, respiratory syncytial virus, rhinoviruses, rotaviruses, Rubella viruses, and varicella-zoster viruses.

The antigens derived from the pathogenic organisms, for example, may be antigens known to elicit an immune response, for which an enhanced immune response is desired, or the antigen may be one that is known to generate a weak response for which an enhanced response is desired. It is also possible that some antigens that did not previously elicit an immune response will become antigenic as a result of the methods of the invention and the phenomenon of hypermutability of cells which contain dominant negative alleles of mismatch repair genes.

The antigens produced by the method of the invention are novel immunogens that may be administered in an appropriate pharmaceutical carrier, such as an adjuvant, for administration to animals as a vaccine. The antigens produced by the methods of the invention may be administered to animals in immunogenic amounts such that an antibody and/or a cell-mediated immune response is elicited. The administration of the antigen produced by the methods of the invention may be as a single dose, or, preferably as a plurality of doses to effect a boosted immune response. The route of administration may be any accepted route of immunization including, for example, oral, intrmuscular, intrperitoneal, subcutaneous, intradermal, intranasal, or transdermal.

Doses for humans can readily be extrapolated from animal studies as taught by Katocs *et al.,* Chapter 27 of REMINGTON'S PHARMACEUTICAL SCIENCES, 18^{th} Edition, Gennaro (Ed.) Mack Publishing Co., Easton, PA, 1990. Immunogenic dosages can be adjusted by one skilled in the art, and may vary depending on several factors, including the age, health, physical condition, weight, type and extent of the disease or disorder of the recipient, frequency of treatment, the nature of concurrent therapy, if required, and the nature and scope of the desired effect(s) (Nies *et al.,* Chapter 3, GOODMAN & GILMAN'S THE PHARMACOLOGICAL BASIS OF THERAPEUTICS, 9th Ed., Hardman *et al.,* Eds., McGraw-Hill, New York, NY. 1996). Typically, an immunogenic amount of the antigens produced by the methods of the invention will be in the range of about 5 to about 100 g.

The antigens of the present invention may be administered as single antigens or may be administered as combinations of antigens. As a non-limiting example, the antigen combinations may be antigens of the same pathogenic organism, or may be antigens of different pathogenic organisms, such that immune responses are elicited to more than one pathogenic organism.

The antigens produced by the methods of the present invention are hypermutated by the methods of the invention which take advantage of the mismatch repair system. The process of MMR, also called mismatch proofreading, is carried out by protein complexes in cells ranging from bacteria to mammalian cells. A MMR gene is a gene that encodes for one of the proteins of such a mismatch repair complex. Although not wanting to be bound by any particular theory of mechanism of action, a MMR complex is believed to detect distortions of the DNA helix resulting from non-complementary pairing of nucleotide bases. The non-complementary base on the newer DNA strand is excised, and the excised base is replaced with the appropriate base, which is complementary to the older DNA strand. In this way, cells eliminate many mutations that occur as a result of mistakes in DNA replication.

Dominant negative alleles cause a MMR defective phenotype even in the presence of a wild-type allele in the same cell. An example of a dominant negative allele of a MMR gene is the human gene *hPMS2-134,* which carries a truncating mutation at codon 134. The mutation causes the product of this gene to abnormally terminate at the position of the 134th amino acid, resulting in a shortened polypeptide containing the N-terminal 133 amino acids. Such a mutation causes an increase in the rate of mutations, which accumulate in cells after DNA replication. Expression of a dominant negative allele of a mismatch repair gene results in impairment of mismatch repair activity, even in the presence of the wild-type allele. Any allele that produces such effect can be used in this invention.

Dominant negative alleles of a MMR gene can be obtained from the cells of humans, animals, yeast, bacteria, or other organisms (Prolla T.A. *et al.* (1994) *Science* 264:1091-1093; Strand *M. et al.* (1993) *Nature* 365:274-276; Su, S.S. *et al.* (1988) *J*. *Biol. Chem.* 263:6829-6835). Such alleles can be identified by screening cells for defective MMR activity. Cells from animals or humans with cancer can be screened for defective mismatch repair. Cells from colon cancer patients may be particularly useful. Genomic DNA, cDNA, or mRNA from any cell encoding a MMR protein can be analyzed for variations from the wild type sequence. Dominant negative alleles of a MMR gene can also be created artificially, for example, by producing variants of the *hPMS2-134* allele or other MMR genes. Various techniques of site-directed mutagenesis can be used. The suitability of such alleles, whether natural or artificial, for use in generating hypermutable cells or animals can be evaluated by testing the mismatch repair activity caused by the allele in the presence of one or more wild-type alleles, to determine if it is a dominant negative allele.

A cell or an animal into which a dominant negative allele of a mismatch repair gene has been introduced will become hypermutable. This means that the spontaneous mutation rate of such cells or animals is elevated compared to cells or animals without such alleles. The degree of elevation of the spontaneous mutation rate can be at least 2-fold, 5-fold, 10-fold, 20-fold, 50-fold, 100-fold, 200-fold, 500-fold, or 1000-fold that of the normal cell or animal. The use of chemical mutagens such as but limited to methane sulfonate, dimethyl sulfonate, 06-methyl benzadine, MNU, ENU, etc. can be used in MMR defective cells to increase the rates an additional 10 to 100 fold that of the MMR deficiency itself.

According to one aspect of the invention, a polynucleotide encoding for a dominant negative form of a MMR protein is introduced into a cell. The gene can be any dominant negative allele encoding a protein, which is part of a MMR complex, for example, *PMS2, PMS1, MLH1,* or *MSH2.* The dominant negative allele can be naturally occurring or made in the laboratory. The polynucleotide can be in the form of genomic DNA, cDNA, RNA, or a chemically synthesized polynucleotide.

The polynucleotide can be cloned into an expression vector containing a constitutively active promoter segment [such as but not limited to CMV, SV40, Elongation Factor (EF) or LTR sequences] or to inducible promoter sequences such as the steroid inducible pIND vector (InVitrogen), tetracycline, or MMTV, where the expression of the dominant negative MMR gene can be regulated. The polynucleotide can be introduced into the cell by transfection.

According to another aspect of the invention, a gene, a set of genes or a chimeric gene encoding for whole or parts of a therapeutic antigen can be transfected into MMR. deficient cell hosts, the cell is grown and screened for clones containing genetically altered genes encoding for antigens with new biochemical features including but not limited to increased antigenicity. MMR defective cells may be of human; primates, mammals, rodent, plant, yeast or of the prokaryotic kingdom.

Transfection is any process whereby a polynucleotide is introduced into a cell. The process of transfection can be carried out in a living animal, e.g., using a vector for gene therapy, or it can be carried out *in vitro, e.g.,* using a suspension of one or more isolated cells in culture. The cell can be any type of eukaryotic cell, including, for example, cells isolated from humans or other primates, mammals or other vertebrates, invertebrates, and single celled organisms such as protozoa, yeast, or bacteria.

In general, transfection will be carried out using a suspension of cells, or a single cell, but other methods can also be applied as long as a sufficient fraction of the treated cells or tissue incorporates the polynucleotide so as to allow transfected cells to be grown and utilized. The protein product of the polynucleotide may be transiently or stably expressed in the cell. Techniques for transfection are well known. Available techniques for introducing polynucleotides include but are not limited to electroporation, transduction, cell fusion, the use of calcium chloride, and packaging of the polynucleotide together with lipid for fusion with the cells of interest. Once a cell has been transfected with the dominant negative MMR gene, the cell can be grown and reproduced in culture. If the transfection is stable, such that the gene is expressed at a consistent level for many cell generations, then a cell line results.

An isolated cell is a cell obtained from a tissue of humans or animals by mechanically separating out individual cells and transferring them to a suitable cell culture medium, either with or without pretreatment of the tissue with enzymes, e.g., collagenase or trypsin. Such isolated cells are typically cultured in the absence of other types of cells. Cells selected for the introduction of a dominant negative allele of a mismatch repair gene may be derived from a eukaryotic organism in the form of a primary cell culture or an immortalized cell line, or may be derived from suspensions of single-celled organisms.

A polynucleotide encoding for a dominant negative form of a MMR protein can be introduced into the genome of an animal by producing a transgenic animal. The animal can be any species for which suitable techniques are available to produce transgenic animals. For example, transgenic animals can be prepared from domestic livestock, e.g., bovine, swine, sheep, goats, horses, etc.; from animals used for the production of recombinant proteins, e.g., bovine, swine, or goats that express a recombinant polypeptide in their milk; or experimental animals for research or product testing, e.g., mice, rats, guinea pigs, hamsters, rabbits, etc. Cell lines that are determined to be MMR defective can then be used as a source for producing genetically altered genes encoding for therapeutic antigens *in vitro* by introducing whole, intact genes and/or chimeric genes encoding for a therapeutic antigen(s) into MMR defective cells from any tissue of the MMR defective animal.

Once a transfected cell line or a colony of transgenic animals has been produced, it can be used to generate new mutations in one or more gene(s) of interest. A gene of interest can be any gene naturally possessed by the cell line or transgenic animal or introduced into the cell line or transgenic animal. An advantage of using such cells or animals to induce mutations is that the cell or animal need not be exposed to mutagenic chemicals or radiation, which may have secondary harmful effects, both on the object of the exposure and on the workers. However, chemical mutagens may be used in combination with MMR deficiency, which renders such mutagens less toxic due to an undetermined mechanism. Hypermutable animals can then be bred and selected for those producing genetically variable cells that may be isolated and cloned to identify new cell lines that are useful for producing structurally altered polypeptides. Once an altered polypeptide is identified, the dominant negative MMR gene allele can be removed by directly knocking out the allele by technologies used by those skilled in the art or by breeding to mates lacking the dominant negative allele to select for offspring with a desired trait and a stable genome. Another alternative is to use a CRE-LOX expression system, whereby the dominant negative allele is spliced from the animal genome once an animal containing a genetically diverse protein profile has been established. Yet another alternative is the use of inducible vectors such as the steroidinduced pIND (InVitrogen) or pMAM (Clonetech) vectors which express exogenous genes in the presence of corticosteroids.

Mutations can be detected by analyzing for alterations in the genotype of the cells or animals, for example by examining the sequence of genomic DNA, cDNA, messenger RNA, or amino acids associated with the gene of interest. Mutations can also be detected by screening for the production of antigenicity. A mutant polypeptide can be detected by identifying alterations in electrophoretic mobility, spectroscopic properties, or other physical or structural characteristics of a protein encoded by a mutant gene. One can also screen for altered function of the protein *in situ,* in isolated form, or in model systems. One can screen for alteration of any property of the cell or animal associated with the function of the gene of interest, such as but not limited to antigenicity.

Examples of mismatch repair proteins and nucleic acid sequences include the following:
PMS2 (mouse) (SEQ ID NO:5),
PMS2 (mouse cDNA) (SEQ ID NO:6),
PMS2 (human) (SEQ ID NO:7),
PMS2 (human cDNA) (SEQ ID NO:8),
PMS1 (human) (SEQ ID NO:9),
PMS 1 (human) (SEQ ID NO:10),
MSH2 (human) (SEQ ID NO:11),
MSH2 (human cDNA) (SEQ ID NO:12),
MLH1 (human) (SEQ ID NO: 13),
MLH1 (human) (SEQ ID NO:14),
hPMS2-134 (human) (SEQ ID NO:15) and
hPMS2-134 (human cDNA) (SEQ ID NO:16)

For further information on the background of the invention the following references may be consulted:
1. John, T.J. (2000) The final stages of the global eradication of polio. *New Engl. J. Med* 14:806-807.
2. Boyce, *T.G. et al.* (2000) Safety and immunogenicity of adjuvanted and unadjuvanted subunit influenza vaccines administered intranasally to healthy adults. *Vaccine* 19:217-226.
3. Hoshino, Y. and A.Z. Kapikian (2000) Rotavirus serotypes: classification and importance in epidemiology, immunity, and vaccine development. *J*. *Health Popul. Nutr.* 18:5-14.
4. Orenstein, W.A. *et al.* (2000) Measles eradication: is it in our future? *Am. J. Public Health* 90:1521-525.
5. Lechmann, M, and T.J. Liang (2000) Vaccine development for hepatitis C. *Semin. Liver Dis.* 20:211-226.
6. Ausiello, *C.M. et al.* (1999) Cell-mediated immune responses in four-year-old children after primary immunization with acellular pertussis vaccines. *Infect. Immun.* 67:4064-4071.
7. Brosstoff, S. (1995) The development and use of T cell receptor peptide vaccines. *Adv. Exp. Med. Biol.* 383:249-254.
8. Oaks, S.C., Jr. V.S. Mitchell, G.W. Pearson, and C.J. Carpenter (1991) MALARIA OBSTACLES AND OPPORTUNITIES, National Academy Press, p. 1, 1991.
9. Anders, R.F. "Vaccines against asexual blood stages of *Plasmodium falciparum"* NEW GENERATION VACCINES, 2^{nd} Ed., pp. 1035-1055,1997.
10. McLeod, *R. et al.* (1995) Immunogenetics in the analysis of resistance to intracellular pathogens. Curr. Opin. Immunol. 7:539-552.
11. Corbel, M.J. (1996) Reasons for instability of bacterial vaccines. *Dev. Biol. Stand.* 87:113-124.
12. Kniskern, P.J. *et al.* (1994) Characterization and evaluation of a recombinant hepatitis B vaccine expressed in yeast defective for N-linked hyperglycosylation. *Vaccine* 12:1021-1025.
13. Kim, K. *et al.* (1994) Conformationally appropriate expression of the *Toxoplasma* antigen SAG1 (p30) in CHO cells. *Infect. Immun.* 62:203-209.
14. Baker, S.M. *et al.* (1995) Male defective in the DNA mismatch repair gene PMS2 exhibit abnormal chromosome synapsis in meiosis. *Cell* 82:309-319.
15. Bronner, C.E. *et al.* (1994) Mutation in the DNA mismatch repair gene homologue *hMLH1* is associated with hereditary non-polyposis colon cancer. *Nature* 368:258-261.
16. de Wind N. *et al.* (1995) Inactivation of the mouse *Msh2* gene results in mismatch repair deficiency, methylation tolerance, hyperrecombination, and predisposition to cancer. *Cell* 82:321-300.
17. Drummond, J.T. *et al.* (1995) Isolation of an hMSH2-p 160 heterodimer that restores mismatch repair to tumor cells. *Science* 268:1909-1912.
18. Modrich, P. (1994) Mismatch repair, genetic stability, and cancer. *Science* 266:1959-1960.
19. Hoang, J.M. *et al.* (1997) BAT-26, an indicator of the replication error phenotype in colorectal cancers and cell lines. *Cancer Res. 15:300-303.*
20. Jiricny, J. and M. Nystrom-Lahti (2000) Mismatch repair defects in cancer. *Curr. Opin. Genet. Dev.* 10:157-161.
21. Nicolaides, N.C. *et al.* (1998) A naturally occurring hPMS2 mutation can confer a dominant negative mutator phenotype. *Mol. Cell. Biol.* 18:1635-1641.
22. Prolla, T.A. *et al.* (1994) MLH1, PMS1, and MSH2 interaction during the initiation of DNA mismatch repair in yeast. *Science* 264:1091-1093.
23. Strand, M. *et al.* (1993) Destabilization of tracts of simple repetitive DNA in yeast by mutations affecting DNA mismatch repair. *Nature* 365:274-276.
24. Su, S.S., R.S. Lahue, K.G. Au, and P. Modrich (1988) Mispair specificity of methyl directed DNA mismatch corrections *in vitro. J. Biol. Chem.* 263:6829-6835.
25. Parsons, *R. et al.* (1993) Hypermutability and mismatch repair deficiency in RER⁺ tumor cells. *Cell* 75:1227-1236.
26. Papadopoulos, *N. et al.* (1993) Mutation of a *mutL* homolog is associated with hereditary colon cancer. *Science* 263:1625-1629.
27. Perucho, M. (1996) Cancer of the microsatellite mutator phenotype. *Biol. Chem.* 377:675-684.
28. Karran P. and R. Hampson (1996) Genomic instability and tolerance to alkylating agents. *Cancer. Surv.* 28:69-85.
29. Palombo, F. *et al.* (1994) Mismatch repair and cancer. *Nature* 36:417.
30. Eshleman J.R. and S.D. Markowitz (1996) Mismatch repair defects in human carcinogenesis. *Hum. Mol. Genet.* 5:1489-494.
31. Liu, *T. et al.* (2000) Microsatellite instability as a predictor of a mutation in a DNA mismatch repair gene in familial colorectal cancer. *Genes Chrom. Cancer* 27:17-25.
32. *Devos et al.* (1983) Molecular cloning of human interleukin-2 cDNA and its expression in E. *coli. Nucl. Acids Res.* 11:4307-4323.
33. Nicolaides, *N.C., et al.* (1995) Genomic organization of the human PMS2 gene family. *Genomics 30:195-206.*
34. Papadopoulos, *N. et al.* (1994) Mutation of a *mutL* homolog in hereditary colon cancer. *Science* 263:1625-1629.
35. Papadopoulos, N., *et al.* (1995) Mutations of GTBP in genetically unstable cells. *Science* 268:1915-1917.
36. Nicolaides, N.C. *et al.* (1997) Interleukin 9: a candidate gene for asthma. *Proc. Natl. Acad. Sci. USA* 94:13175-13180.
37. Grasso, *L. et al.* (1998) Molecular analysis of human interleukin-9 receptor transcripts in peripheral blood mononuclear cells. Identification of a splice variant encoding for a nonfunctional cell surface receptor. *J. Biol. Chem.* 273:24016-24024.
38. Sela, M. (2000) Structural components responsible for peptide antigenicity. *Appl. Biochem. Biotechnol.* 83:63-70.
39. Galio, *L. et al.* (1999) ATP hydrolysis-dependent formation of a dynamic ternary nucleoprotein complex with *MutS* and *MutL. Nucl. Acids Res.* 27:2325-2331.
40. Spampinato, C. and P. Modrich (2000) The *MutL* ATPase is required for mismatch repair. *J. Biol. Chem.* 275:9863-9869.

The above disclosure generally describes the present invention. A more complete understanding can be obtained by reference to the following specific examples which are provided herein for purposes of illustration only, and are not intended to limit the scope of the invention.

### EXAMPLE 1: Stable expression of dominant negative MMR genes in cells results in widespread mutations of a reporter gene and its encoded polypeptide.

Expression of a dominant negative allele in an otherwise MMR proficient cell could render these host cells MMR deficient (Nicolaides, *N.C. et al.* (1998) *Mol. Cell. Biol.* 18:1635-1641). The creation of MMR deficient cells can lead to the generation of genetic alterations throughout the entire genome of a host organisms offspring, yielding a population of genetically altered offspring or siblings that may produce biochemicals with altered properties. This patent application teaches of the use of dominant negative MMR genes in antigen-producing cells, including but not limited to rodent, human, primate, yeast, insect, and prokaryotic cells producing proteins that may serve as therapeutic antigens for vaccination. The cell expression systems described above that are used to produce antigens are well known by those skilled in the art of vaccine therapeutics.

To demonstrate the ability to create MMR defective mammalian cells using dominant negative alleles of MMR genes, we first transfected a MMR proficient rodent cell line with an expression vector containing the human the previously published dominant negative PMS2 mutant referred herein as PMS 134 (cell line referred to as TKPMS 134), or with no insert (cell line referred to as TKvec). A fragment containing the PMS 134 cDNA was cloned into the pEF expression vector which contains the constitutively active elongation factor promoter along with the neomycin resistance gene as selectable marker. The results showed that the PMS 134 mutant could exert a robust dominant negative effect, resulting in biochemical and genetic manifestations of MMR deficiency. A brief description of the methods are provided below.

A hallmark of MMR deficiency is the generation of unstable microsatellite repeats in the genome of host cells. This phenotype is referred to as microsatellite instability (MI). MI consists of deletions and/or insertions within repetitive mono-, di- and/or tri nucleotide repetitive sequences throughout the entire genome of a host cell. Extensive genetic analysis eukaryotic cells have found that the only biochemical defect that is capable of producing MI is defective MMR. In light of this unique feature that defective MMR has on promoting MI, it is now used as a biochemical marker to survey for lack of MMR activity within host cells.

A method used to detect MMR deficiency in eukaryotic cells is to employ a reporter gene that has a polynucleotide repeat inserted within the coding region that disrupts its reading frame due to a frame shift. In the case where MMR is defective, the reporter gene will acquire random mutations (i.e. insertions and/or deletions) within the polynucelotide repeat yielding clones that contain a functional reporter gene. An example of the ability to alter desired genes via defective MMR comes from experiments using Syrian Hamster fibroblasts (TK) cells (described above), where a mammalian expression construct containing a defective β-galactosidase gene (referred to as pCAR-OF) was transfected into TKPMS134 or TKvect cells as described above. The pCAR-OF vector consists of a β-galactosidase gene containing a 29-basepair poly-CA tract inserted at the 5' end of its coding region, which causes the wild-type reading frame to shift out-of-frame. This chimeric gene is cloned into the pCEP4, which contains the constitutively cytomegalovirus (CMV) promoter upstream of the cloning site and also contains the hygromycin-resistance (HYG) gene that allows for selection of cells containing this vector. The pCAR-OF reporter cannot generate β-galactosidase activity unless a frame-restoring mutation,(i.e., insertion or deletion) arises following transfection into a host. Another reporter vector called pCAR-IF contains a β-galactosidase in which a 27-bp poly-CA repeat was cloned into the same site as the pCAR-OF gene, but it is biologically active because the removal of a single repeat restores the open reading frame and produces a functional chimeric β-galactosidase polypeptide (not shown). The pCAR vectors also contain the neomycin resistance gene as selectable marker. In these proof-of-concept studies, TKPMS134 and TKvect cells were transfected with the pCAR-OF reporter vector and selected for 17 days in neomycin plus hygromycin selection medium. After the 17^{th} day, resistant colonies were stained for β-galactosidase production to determine the number of clones containing a genetically altered β-galactosidase gene. All conditions produced a relatively equal number of neomycin/hygromycin resistant cells, however, only the cells expressing the PMS 134 dominant negative allele (TKPMS 134) contained a subset of clones that were positive for β-galactosidase activity (Table 1). This result was also observed using a similar experimental strategy with a MMR proficient human cell line (data not shown). Table 1 shows the data from these experiments, where cell colonies were stained *in situ* for β-galactosidase activity and scored for activity. Cells were scored positive if the colonies turned blue in the presence of X-gal substrate and scored negative if colonies remained white. Analysis of triplicate experiments showed a significant increase in the number of β-galactosidase positive cells in the TKPMS 134 cultures, while no β-galactosidase cells were seen in the control TKvect cells.

**Table 1.**

| **Number of TKPMS134 and TKvect cells containing functional β-galactosidase gene as a result of MMR deficiency.** | | | |
|---|---|---|---|
| **Cells** | **White Colonies** | **Blue Colonies** | **% Clones with altered B-gal** |
| TKvect | 65 +/- 9 | 0 | 0/65 = 0% |
| TKPMS134 | 40 +/- 12 | 28 +/- 4 | 28/68 = 41% |

**Table 1**. β-galactosidase expression of HBvec, HBPMS2 and HB134 cells transfected with pCAR-OF reporter vectors. Cells were transfected with the pCAR-OF β-galactosidase reporter plasmid. Transfected cells were selected in hygromycin and G418, expanded and stained with X-gal solution to measure for β-galactosidase activity (blue colored cells). 3 plates each were analyzed by microscopy. The results below represent the mean +/- standard deviation of these experiments.

TKPMS134/pCAR-OF clones that were pooled and expanded also showed a number of cells that contained a functional β-galactosidase gene. No β-galactosidase positive cells were observed in TKvect cells transfected with the pCAR-OF vector. These data are shown in Figure 1 where the dark staining in panel B represent β-galactosidase positive cells present in the TKPMS134/pCAR-OF cultures while none are found in the TKvect cells grown under similar conditions (panel A). These data demonstrate the ability of dominant negative alleles of MMR genes to generate *in vivo* gene alterations, which allows for the rapid screening of clones with altered polypeptides exhibiting new biochemical features.

To confirm that alterations within the nucleotide sequences of the β-galactosidase gene was indeed responsible for the *in vivo* β-galactosidase activity present in TKPMS134 clones, RNA was isolated from TKPMS134/pCAR-OF and TKvect/pCAR-OF and the β-galactosidase mRNA primary structure was examined by reverse transcriptase polymerase chain reaction (RT-PCR) amplification and sequencing. Sequence analysis of β-galactosidase message from TKvect cells found no structural alterations in the input gene sequence. Analysis of the β-galactosidase message from TKPMS134 cells found several changes within the coding sequences of the gene. These sequence alterations included insertion and deletions of the poly-CA tract in the amino terminus as expected. Other alterations included insertions of sequences outside of the poly-CA repeat as well as a series of single base alterations (transversions and transitions) contained throughout the length of the gene.

### In situ X-gal staining

For *in situ* analysis, 100,000 cells are harvested and fixed in 1% gluteraldehyde, washed in phosphate buffered saline solution and incubated in 1 ml of X-gal substrate solution [0.15 M NaCl, 1 mM MgCl₂, 3.3 mM K₄Fe(CN)₆, 3.3 mM K₃Fe(CN)₆, 0.2% X-Gal] in 24 well plates for 2 hours at 37°C. Reactions are stopped in 500 mM sodium bicarbonate solution and transferred to microscope slides for analysis. Three plates each are counted for blue (β-galactosidase positive cells) or white (β-galactosidase negative cells) to assess for MMR inactivation. Table 1 shows the results from these studies.

### EXAMPLE 2: Generation of an expression cassette for screening of structurally altered polypeptides in MMR defective cells.

In order to produce recombinant proteins for screening of highly antigenic polypeptides, a fusion gene cassette was engineered that encodes for a secreted polypeptide containing a six polyhistidine domain at the C-terminus, which is useful for purification. This gene cassette is referred to as sec-hist. This gene was constructed by PCR using DNA from the pUC18 plasmid as template. The sense primers contained nucleotide sequences corresponding to the leader sequence of human interleukin-2 (ref 32), which has been found to produce robust amounts of secreted polypeptides from TK cells (personal observation). This domain was introduced at the 5' end of the pUC18 polylinker. Antisense primers containing nucleotide sequences encoding for 6 histidines were used to position these residues at the 3' end of the pUC18 polylinker. The nucleotide sequence of these primers are listed below.

### SENSE Primer:

5' *aagctt*ccatgtacaggatgcaactcctgtcttgcattgcactaagtcttgcacttgtcacaaacagtgcaCAAAAGCTG GAGCTC-3' (SEQ ID NO:1)

The italic sequence represents a HindIII site for subcloning. The underlined sequence represents leader sequence from the human IL-2. Sequence in capital letters represents sequence from the start of the polylinker region of pUC18.

### ANTISENSE Primer:

5' *ccggataccc*tactagtggtgatggtgatggtgGCTTGATATCGAATTCCTG-3' *(S*EQ ID NO:2)

The italic sequence represents a HindIII site for subcloning. The underlined sequence represents 6 codons encoding for histidine residues followed by 2 termination codons. Sequence in capital letters represents sequence to the 3' end of the pUC18 polylinker.

Amplified products were obtained using buffer conditions as previously described. Amplification reactions were carried out at 94°C for 30 sec, 52°C for 2 min, and 72°C for 2 min for 25 cycles. Products were run on 1% agarose gels containing ethidium bromide, and products of the expected molecular weight were excised and purified by Gene Clean (Bio101). Products were then cloned into T-tailed vectors (InVitrogen) as suggested by the manufacturer. Recombinant clones were analyzed by restriction analysis and by DNA sequencing. Several clones contained fragments with the expected genomic sequence. The parental clone is referred to as TAsec-hist.

A schematic diagram of the sec-hist fusion protein is shown in Figure 3A In order to generate TK cells that secrete the sec-hist polypeptide, the TAsec-hist plasmid is digested with HindIII to release the sec-hist insert. The insert was cloned into the unique HindIII site of the pCEP4 mammalian expression vector, which also contains the Hygromycin resistance gene as selectable marker. Recombinant clones were analyzed by restriction digest and sequencing to assure the authenticity of the construct.

Inserts can now be designed via PCR or direct cloning using the restricition sites contained within the polylinker (see Figure 3B).

Recombinant pCEPsec-hist plasmid will then be transfected into TK cells as previously described using cationic lipids. Cells will be cotransfected along with the pEFPMS134, which is a mammalian expression vector containing the PMS134 dominant negative MMR gene allele under control of the constitutive elongation factor (EF) promoter. This vector contains the neomycin resistance gene and allows for double selection of TK cells for both the sec-hist and pEFPMS134 vectors. TK cells will also be cotransfected with the sec-hist and pEF empty vector as a control.

Cells are co-selected for 14 days in 0.6 mg/ml G418 and 0.8mg/ml hygromycin B (these concentrations have been previously determined for double transfection of TK cells). After 14 days, macroscopic colonies will be isolated and subcloned into 24 well dishes (Nunc) as 1 ml cultures. Clones will then be analyzed for secreted sec-hist protein using both ELISA and western blot analysis of conditioned supernatants from sec-hist/pEFPMS 134, sec-hist/pEFempty vector, and parental TK cells. A monoclonal anti-HIS antibody (Santa Cruz), which has been successfully used for other western and ELISA studies, will be used for both assays. Analysis of PMS134 expression will be determined by western blot using a PMS2-specific polyclonal antibody (Morphotek, personal communication).

ELISA will be performed on conditioned medium (CM) from TK cells transfected with pCEP4sec-hist to screen for high producers of the sec-hist polypeptide. ELISAs are carried out as follows. Twohundred microliter aliquots of conditioned medium are taken from pCEP4sec-hist transfected and control cells. Aliquots are placed into 1.5ml eppendorf tubes and centrifuged at 14,000Xg for 3 minutes to pellet cell debris.
Supernates are then collected and 50 µls are placed into triplicate wells of a 96-well polystyrene microtiter plate (Nunc). Plates are incubated at room temperature for 4 hours, washed twice with 200 µls of 1X Phosphate Buffered Saline (PBS) solution, pH 7.0 (Life Technologies), and blocked with 100 µls of 5%milk in 1X PBS for 1 hour. Plates are then incubated with a monoclonal anti-HIS antibody (diluted 1:1000 in 1X PBS) (Santa Cruz) for 2 hours at room temperature and then washed twice with 200 µls of 1X PBS, and probed with an anti-mouse-horse radish peroxidase (HRP) conjugated secondary antibody diluted 1:3000 in PBS. Plates are then incubated at room temperature for 1 hour, washed three times with 200 µls of 1X PBS and incubated with TMB substrate (BioRad) for 15-30 minutes. After incubation is completed, reactions are stopped using 0.1N H₂SO₄ and plates are read using a BioRad microplate reader at 415nm. Clones are determined to be positive for secreted sec-hist if expected cells are found to produce a significant signal over control cells. Conditioned medium from positive cultures will then be analyzed by western blot using the anti-HIS antibody as probe to confirm ELISA data.

Western blot analysis will be carried out as follows. Briefly, 50µls of CM or 50,000 cells from each culture is directly lysed in 2X lysis buffer (60 mM Tris, pH 6.8, 2% SDS, 10% glycerol, 0.1 M 2-mercaptoethanol, 0.001% bromophenol blue) and samples are boiled for 5 minutes. Lysate proteins are separated by electrophoresis on 4-20% Tris glycine gels (Novex) and then electroblotted onto Immobilon-P (Millipore) in 48 mM Tris base, 40 mM glycine, 0.0375% SDS, 20% methanol and blocked overnight at 4°C in Tris-buffered saline plus 0.05% Tween-20 and 5% condensed milk. Filters are then probed with an antibody generated against the PMS 134 or the polyHIS tag (Santa Cruz), followed by a secondary HRP-conjugated antibody. After incubation with the secondary antibody, blots are developed using chemiluminescence (Pierce) and exposed to film to determine PMS134 and sec-hist expression. Clones exhibiting expression of both genes will then be used in experiments described above.

A potential technical problem may exist in expressing the sec-hist protein due to toxicity that it may have on the growth of TK cells. If the production of no or low amounts of sec-hist polypeptide is found to occur in the above analysis, a HindIII sec-hist fragment from the TAsec-hist plasmid will be subcloned into the unique HindIII site of the pIND/V5 steroid inducible vector (Invitrogen). This vector has been found to produce robust protein expression in TK cells upon in steroid induction. This application teaches the use of employing an inducible vector containing the sec-hist expression cassette to express polypeptides in TK cells that may be toxic under constitutively expressed conditions.
Cells that are found to co-express the PMS134 and the sec-hist genes or the control cell expressing the pEF empty vector and the sec-hist gene are cultured under high growth conditions in media containing neomycin, hygromycin and vitamins, which has been shown to increase the doubling time of TK cells and enhance the genetic alteration of β-galactosidase reporter plasmids *in vivo* (data not shown). Briefly, cells are grown in vitamin enriched medium for 20 doublings (~17 days), a time at which it has been found that 20-40% of clones contain sequence alterations within a particular genetic locus. After selection, cells will be subcloned in 96-well microtiter plates by limiting dilutions. Clones will be grown for 5 days in the presence of neomycin/hygromycin-free medium containing heat inactivated serum to remove complement for *in vitro* antigenic assays that will be

The sec-hist expression vector cassette can also be transfected into cell lines that are "naturally" defective for MMR such as the human cell lines derived from colon cancer tumors such as but not limited to HCT116 and DLD-1. The vector can be in the constitutive backbone pCEP4 or under control of the steroid-inducible vectors pIND or pMAM.

The techniques described above teach us the use of producing structurally altered antigens from mammalian cells to ensure proper folding or post-translation modifications of the polypeptide. This approach gives an advantage over others that employ the use of prokaryotic, yeast or baculovirus produced antigens that have been found to produce weak antigenic responses due to misfolding or improper post-translational modifications.

### EXAMPLE 3: Screening strategy to identify cell clones producing highly antigenic polypeptides.

In order to identify antigenic polypeptides produced by TKPMS 134 cell clones, the following *in vitro* assays will be performed.

First, the lymphocyte stimulatory activity of sec-hist polypeptides will be measured by adding CM of TKsec-hist cells with or without the PMS 134 to lymphocytes derived from naive or whole antigen exposed Balb/C mice. Briefly, 2 mice will be infected with whole antigen in the presence of Freund's Complete Adjuvant by subcutaneous injection in the tail with a 100-µl 1/1 mixture of complete Freund's adjuvant (CFA) (Difco). Two subcutaneous boosts will be performed with the same quantity of antigen, mixed 1/1 with incomplete Freund's adjuvant (Difco), after 2 and 4 weeks. Two control mice will receive adjuvant alone. Mice are sacrificed 5 days after the second boost (at day 33). Peripheral blood mononuclear cells (PBMCs) from whole blood and splenocytes from spleens of will be harvested following the previously described procedures (Nicolaides, *N.C. et al.* (1997) *Proc. Natl. Acad. Sci. USA* 94:13175-13180). For splenocyte assays, whole spleens are pressed through sterile wire mesh into RPMI medium (Life Technology). Next, cells are washed twice in RPMI and incubated for 10 minutes in RBC lysis buffer. Cells are then washed again and resuspended at 1 X 10⁵ cells/ml in RPMI-1640 medium plus 10% heat inactivated fetal bovine serum. One hundred microliters of cells are aliquoted into twenty 96-well titer flat bottom plates.

For PBMC isolation, whole blood is isolated by eye puncture and collected into vacutainer tubes containing EDTA. An equal volume of PBS (Mg²⁺/Ca²⁺-free) is added to whole blood. PBMCs are isolated by centrifugation over Ficoll-Paque gradients (Pharmacia Biotech 17-1440-02). Purified cells are seeded at 1 X 10⁵ cells/ml in RPMI 1640 containing 10% heat-inactivated fetal bovine serum (Life Technologies, Inc.) and 100µls are plated in 96 well flat bottom microtiter plates and incubated at 37 °C in 5% CO₂.

To measure for T-cell activation, PBMCs and splenocytes from primed and non-primed mice are incubated with 10% conditioned medium (CM) from TKsec-hist cells with or without the PMS134 gene. 5 µg/ml of concavalinA (ConA) is used as a positive control for splenocyte culture assays, while 5 µg/ml phorbol 12-myristate 13-acetate and 1 µg/ml phytohemagglutinin (Sigma) are used as a positive control for PBMC cultures. CM from parental TK cells grown in the presence of RPMI with 10% heat inactivated medium will be used as negative control. Previous studies using CM from TK cells have found no stimulatory activity to be produced on PBMCs or splenocytes (N. Nicolaides, personal observation). Cultures are incubated at 37 °C in 5% CO₂ for 6 days and scored for antigenic activity as determined by proliferation assay. Proliferation is assayed using a modified protocol of the acid phosphatase assay as described (Grasso, L. *et al.* (1998) *J. Biol. Chem.* 273:24016-24024). Briefly, 50 µls of a buffer containing 0.1 Msodium acetate (pH 5.5), 0.1% Triton X-100, and 10 mM *p*-nitrophenyl phosphate (Sigma 104 phosphatase substrate) is added directly to each well containing 0.2 ml of growth medium and incubated for 1.5 h atroom temperature. Reactions are terminated by the addition of 0.05 N sodium hydroxide and quantified by absorbance at 410 nmusing a BioRad plate reader. Data is represented as a stimulation index (SI), which is the proliferation of experimental data points divided by the mean of 10 aliquots of CM from TK parental cells. All experiments will be performed in at least triplicate.

It is expected that several TKsec-hist clones co-expressing the PMS 134 protein will be found to have an enhanced antigenicity on PBMCs and/or splenocytes due to conformational changes that will occur within the coding region of the target antigen. These changes may form secondary domains that serve as T and/or B cell epitopes and in turn are responsible for stimulating their respective activation. Clones that reproducibly produce enhanced antigenic activation will be sequenced to confirm and identify that a structural alteration(s) has indeed occurred within the coding region of the gene. Sequence data may also shed additional light into the importance of critical domains within this candidate vaccine polypeptide for additional rounds of alteration that may lead to the creation of a super-antigen that may serve as a potent vaccine.

Sequence analysis of clones will be performed as follows. First positive clones will be expanded from 96-well plates to 24-well plates. Confluent wells will be expanded and cells will be harvested for RNA extraction. RNA extraction and reverse transcription will be carried out using the Trizol method as previously described (Nicolaides, N.C. *et al.* (1997) *Proc. Natl. Acad. Sci. USA* 94:13175-13180; Grasso, L. *et al.* (1998) *J. Biol. Chem.* 273:24016-24024). Reverse transcription will be carried out using Superscript II (Life Technology) as previously described (Nicolaides, N.C. *et al.* (1998) *Mol. Cell. Biol.* 18:1635-I641). cDNAs will be amplified to isolate the sec-hist transcript using the sense primer: 5'-catgtacaggatgcaactcctg-3' (SEQ ID NO:3), which is located at the IL-2 leader sequence site (see Figure 3), and the antisense primer: 5'-tactagtggtgatggtgatggtg-3' (SEQ ID NO:4), which is located at the C-terminal polyhis site. Amplification is carried out at 94°C for 30 sec, 52°C for 2 min, 72°C for 2 min for 30 cycles. Reactions are analyzed on agarose gels. If products of the expected molecular weight are generated then samples will be cleaned using the QIAquick PCR template kit (Qiagen) to remove PCR amplimers and sequenced using the following primers that cover the entire coding region of the sec-hist gene. Clones are then sequenced using primers specific to the gene encoding the antigen.

Clones producing genetically altered sec-hist polypeptides will then be expanded into T-75 flasks to a density that will enable for the sufficient production of secreted sec-hist polypeptide in the CM. Conditioned medium containing the sec-hist polypeptide is then collected, and centrifuged at 3,500Xg for 10 minutes to remove cellular debris. CM is then loaded onto a 10ml- HiTrap Nickel column following the manufacturer's protocol (Pharmacia). After absorption and washing, the column is treated with 200 mM imidazole to elute the fusion protein. Recovered polypeptides are then analyzed by SDS-PAGE using 4-12% NuPAGE gels (Novex) and silver stained following the manufacturer's protocol (Novex). Due to the random nature of defective MMR, the possibility exists that sec-hist alleles may be generated by clones producing enhanced antigenic polypeptides, which contain a nonsense or frame-shift mutation, therefore forming polypeptides lacking a polyHIS C-terminus. If a nonsense or frameshift mutation does occur in clones producing polypeptides with increased antigenicity, then the new allele is reengineered via PCR to contain a polyHIS tag at the C-terminus, and this new fusion protein will be rescreened as above.

Purified polypeptides will be rescreened at a final concentration of 10 µg/ml in the *in vitro* assays described above to confirm that the antigenic activity is indeed coming from the sec-hist protein. TK cells producing altered antigens with enhanced activity on both PBMCs and splenocytes will then be tested along with the non-altered sec-hist protein *in vivo* in Balb/C mice for the ability to illicit an immune response in the absence of adjuvant.
A schematic diagram outlining the screening procedure is given in Figure 4.

### EXAMPLE 4: Screening strategy to identify cell clones producing highly immunogenic antigens.

To test the immunogenic potential that the altered polypeptides identified from EXAMPLE 3 have *in vivo,* Balb/C mice will be injected with the 6 most antigenic polypeptides and the wild type sec-hist polypeptide produced from TKEFempty/sec-hist cells in the absence of adjuvant. Briefly, mice will be immunized with 30 µgs of purified sec-hist protein in sterile phosphate buffered saline (PBS) without adjuvant by subcutaneous injection in the tail. One group of mice will receive a 100µls of a mixture of polypeptides diluted 1/1 in complete Freund's adjuvant (CFA) (Difco) as a positive control. Two subcutaneous boosts will be performed with the same quantity of antigen, and the mouse receiving the polypeptide mixture with adjuvant will be boosted with a 1/1 mixture with incomplete Freund's adjuvant (Difco), at 2 and 4 weeks after the initial injection. Mice will be bled 5 and 15 days after the second boost (at day 33) to measure for antigen titers. Control mice will receive PBS alone. Before the start of immunization, a prebleed will be obtained from each mouse.

Immune responses will be measured by screening for whole Ig and sec-hist specific antibody titers by ELISA following the protocol described above. For antigen specific antisera titers, 96-well plates will be coated with a 50uls of a solution containing 5µg/ml each antigen used for vaccination in PBS. Plates will then be probed with serial dilutions of prebleeds, 5 day and 15 day bleeds. Detection of antibodies will be done using a sheep anti-mouse-HRP conjugated secondary antibody followed by incubation with TMB substrate as described above.

Success of the strategy proposed in this program will be demonstrated with the generation of antisera from mice immunized with altered sec-hist protein that is able to cross react with the native sec-hist protein. If no titers are found in the samples without adjuvant, mice will be administrated antigen in complete Freund's Adjuvant and titers analyzed 14 days later, followed by immunization in Incomplete Freund's at day 17 if no titers are found and analyzed at day 31.

A potential problem that may occur with the outlined strategy is that antibody titers may be generated against the sec-polyhistidine fusion domain. To determine if antisera is able to identify authentic sec-his polypeptides, the antigen lacking the polyhistidine domain will be generated by *in vitro* transcription-translation (TNT) in the presence of radiolabelled methionine. A template containing the sec-hist polypeptide will also be made and the encoded protein used as a control. Templates for the TNT reactions will be generated by PCR as described (Nicolaides, *N.C. et al.* (1998) *Mol. Cell.* Biol. 18:1635-1641). Briefly, the TAsec-hist plasmid will be used as template to amplify gene segments that encode for the untqagged antigen or the sec-hist tagged protein.

Translated polypeptides are first analyzed by SDS-PAGE gel electrophoresis and autoradiography to ensure that the polypeptide with the expected molecular weight are synthesized. Proteins are then immunoprecipitated using antiserum from vaccinated mice to determine if these antibodies recognize authentic antigen sequences. Briefly, immunoprecipitations are performed on *in vitro*-translated proteins by mixing the translation reaction mixtures with 100µls of mouse antiserum or 1 µg of a HIS-specific monoclonal antibody (MAB) (Santa Cruz) in 400 µls of EBC buffer (50 mM Tris [pH 7.5], 0.1 M NaCl, 0.5% Nonidet P-40). After incubation for 1 h at 4°C, protein A-Sepharose (Sigma) is added to a final concentration of 10% and the reaction mixtures are incubated at 4°C for 1 h. Proteins bound to protein A are washed five times in EBC and separated by electrophoresis on 4-20% Tris-glycine gradient gels, which are then dried and autoradiographed.

If antisera are able to react with authentic sequences lacking HIS residues, the data from these studies will be continued in further preclinical animal studies.

### Discussion

The initial steps of MMR are dependent on two protein complexes, called MutSα and MutLα. The use of dominant negative MMR alleles are able to perturb the formation of these complexes with downstream biochemicals involved in the excision and polymerization of nucleotides comprising the "corrected" nucleotides. Examples from this application show the ability of a truncated MMR allele (PMS134 is capable of blocking MMR resulting in a hypermutable cell line that gains genetic alterations throughout its entire genome per cell division. Once a cell line is produced that contains genetic alterations within genes encoding for an antigen, it is desirable to restore the genomic integrity of the cell host. This can be achieved by the use of inducible vectors whereby dominant negative MMR genes are cloned into such vectors, introduced into antigen-producing cells and the cells are cultured in the presence of inducer molecules and/or conditions. Inducible vectors include but are not limited to chemical regulated promoters such as the steroid inducible MMTV, tetracycline regulated promoters, temperature sensitive MMR gene alleles, and temperature sensitive promoters.

The results described above lead to several conclusions. First, expression of PMS 134 results in an increase in microsattelite instability in TK cells. That this elevated microsattelite instability is due to MMR deficiency was proven by evaluation of extracts from stably transduced cells and stability of a tract contained within the pCAR-OF vector. The expression of PMS134 results in a polar defect in MMR, which was only observed using heteroduplexes designed to test repair from the 5' direction (no significant defect in repair from the 3' direction was observed in the same extracts). Interestingly, cells deficient in hMLH1 also have a polar defect in MMR, but in this case preferentially affecting repair from the 3' direction. It is known from previous studies in both prokaryotes and eukaryotes that the separate enzymatic components mediate repair from the two different directions. These results strongly suggest a model in which 5' repair is primarily dependent on hPMS2 while 3' repair is primarily dependent on hMLH1. It is easy to envision how the dimeric complex between PMS2 and MLH1 might set up this directionality. The combined results also demonstrate that a defect in directional MMR is sufficient to produce a MMR defective phenotype and suggests that any MMR gene allele is useful to produce genetically altered TK cells, or a cell line that is producing antigenic gene products. Moreover, the use of such MMR alleles will be useful for generating genetically altered polypeptides with altered structures as effective vaccine agents.

This application also teaches us that ANY method used to block MMR can be performed to generate hypermutablility in an antigen-producing cell that can lead to genetically altered proteins with enhanced biochemical features such as but not limited to increased antigenicity, increased immunogenicity, and enhanced pharmacokinetic profiles.

The blockade of MMR in such cells can be through the use of dominant negative MMR gene alleles from any species including bacteria, yeast, protozoa, insects, rodents, primates, mammalian cells, and man. Blockade of MMR can also be generated through the use of antisense RNA or deoxynucleotides directed to any of the genes involved in the MMR biochemical pathway. Blockade of MMR can be through the use of polypeptides that interfere with subunits of the MMR complex including but not limited to antibodies. Finally, the blockade of MMR may be through the use chemicals such as but not limited to nonhydrolyzable ATP analogs, which have been shown to block MMR (Galio, L. *et al.* (1999) *Nucl. Acids Res.* 27:2325-2331; Spampinato, C. and P. Modrich (2000) *J. Biol. Chem.* 275:9863-9869).

## Claims

1. A method for making a hypermutated antigen, comprising introducing into an isolated mammalian cell that expresses a preselected antigen a polynucleotide comprising a dominant negative allele of a mismatch repair gene, wherein said hypermutated antigen comprises increased antigenicity or increased immunogenicity relative to said preselected antigen.

2. The method of claim 1, wherein the mismatch repair gene is PMS2, MLH1, PMS1 or MSH2.

3. A method according to claim 2, wherein the mismatch repair gene is PMS2 and the allele comprises a truncation mutation.

4. A method according to any of claims 1 to 3, wherein said introduction of said polynucleotide is in the presence of at least one DNA mutagen.

5. A method for making randomly altered forms of a secreted antigen, comprising introducing a polynucleotide encoding a tagged antigen into a mismatch repair defective cell.

6. A method according to claim 5, wherein said tagged antigen is screened for increased antigenicity.

7. A method according to claim 5, wherein said tagged antigen is screened for increased immunogenicity.

8. A method according to any of claims 5 to 7, wherein the cells are naturally mismatch repair defective.

9. A method of producing a mutated antigen in a reversibly unstable cell, comprising
introducing into a cell containing a preselected antigen of interest, an inducible expression vector comprising a polynucleotide encoding a dominant negative allele of a mismatch repair gene;
inducing said cell to express said dominant negative mismatch repair gene; and
detecting increased antigenicity or increased immunogenicity of said mutated antigen relative to said preselected antigen.

10. A method according to claim 9, wherein said preselected antigen of interest is encoded on a polynucleotide previously transfected into said cell.

11. A method according to claim 9 or 10, comprising ceasing induction of said dominant negative allele of a mismatch repair gene, thereby stabilizing said cell.

## Patentansprüche

1. Ein Verfahren, zur Herstellung eines hypermutierten Antigens, enthaltend den Schritt des Einfügens in eine isolierte Mammalia-Zelle, die für ein vorausgewähltes Antigen exprimiert, eines Polynukleotides, das ein dominantes negatives Allel eines Mismatchrepairgens enthält, worin dieses hypermutierte Antigen eine erhöhte Antigenizität oder eine erhöhte Immunogenizität bezogen auf dieses vorausgewählte Antigen aufweist.

2. Das verfahren nach Anspruch 1, worin dieses Mismatchrepairgen PMS2, MLH1, PMS1 oder MSH2 ist.

3. Ein Verfahren nach Anspruch 2, worin das Mismatchrepairgen PMS2 ist und das Allel eine Trunkierungsmutation aufweist.

4. Ein Verfahren nach irgendeinem der Ansprüche 1 bis 3, worin dieses Einfügen von diesem Polynukleotid in der Präsenz von mindestens einem DNA-Mutagen vollzogen wird.

5. Ein Verfahren zur Herstellung zufällig geänderter Formen von einem sekretierten Antigen, enthaltend den Schritt des Einfügens eines Polynukleotids, das ein makiertes Antigen in eine Mismatchrepair-Defektzelle codiert.

6. Ein Verfahren nach Anspruch 5, worin dieses makierte Antigen für eine erhöhte Antigenizität gescreent ist.

7. Ein Verfahren nach Anspruch 5, worin dieses makierte Antigen für eine erhöhte Immunogenizicät gescreent ist.

8. Ein Verfahren nach irgendeinem der Ansprüche 5 bis 7, worin diese Zellen natürliche Mismatchrepair-Defekte sind.

9. Ein Verfahren zur Herstellung eines mutierten Antigens in einer reversiblen unstabilen zelle, mit den Schritten
Einfügen in eine Zelle, die ein von Interesse seiendes preselektierendes Antigen enthält, von einem induzierbaren Expressionsvektor, der ein Polynukleotid enthält, das ein dominantes negatives Allel von einem Mismatchrepairgen codiert;
Einfügen dieser Zelle, um dieses dominante negative Mismatchrepairgen auszuprägen; und
detektieren dieser erhöhten Antigenizität oder dieser erhöhten Immunogenizität von diesem mutierten Antigen relativ zu diesem präselektierten Antigen.

10. Ein Verfahren nach Anspruch 9, worin dieses interessierende voraus selektierte Antigen auf einem Polynukleotid codiert ist, das vorher in diese zelle transfektiert worden ist.

11. Ein Verfahren nach Anspruch 9 oder 10, enthaltend den Verfahrensschritt des Beendens des Einfügens von diesen dominanten negativen Allelen von einem Mismatchrepairgen, wodurch diese Zelle stabilisiert wird.

## Revendications

1. Procédé pour produire un antigène hypermuté, comprenant l'introduction une isolée de qui exprime un antigène présélectionné, un polynucléotide comprenant un allèle négatif dominant d'un gène de réparation de mésappariement, dans lequel ledit antigène hypermuté comprend une antigénicité ou immunogénicité accrue par rapport au dit antigène présélectionné.

2. Procédé suivant la revendication 1, dans le gène de réparation de mésappariement est PMS2, MLH1, PMS1 ou MSH2.

3. Procédé suivant la revendication 2, dans lequel le gène de réparation de mésappariement est PMS2 et l'allèle comprend une mutation par troncature.

4. Procédé suivant l'une quelconque des revendications 1 à 3, dans lequel l'introduction dudit polynucléotide est effectuée en présence d'au moins un mutagène dADN.

5. Procédé pour produire des formes altérées au hasard d'un antigène sécrété, comprenant l'introduction d'un antigène marqué dans une cellule déficiente en moyen de réparation de mésappariement.

6. Procédé suivant la revendication dans lequel ledit antigène marqué est criblé pour un accroissement d'antigénicité.

7. Procédé suivant la revendication 5, dans lequel ledit antigène marqué est criblé pour un accroissement d'immunogénicité.

8. Procédé suivant l'une quelconque des revendications 5 à 7, dans lequel les cellules sont naturellement défficientes en moyen de réparation de mésappariement.

9. Procédé pour produire un antigène muté dans une cellule réversblement instable, comprenant
l'introduction, dans une cellule contenant un antigène présélectionné présentant un intérêt, un vecteur d'expression inductible comprenant un polynucléotide codant pour un allèle négatif dominant d'un gène de réparation de mésappariement ;
l'induction de ladite cellule pour exprimer ledit gène de réparation de mésappariement; et
la détection d'une antigénicité accrue ou d'une immunogénicité accrue dudit antigène muté par rapport audit antigène présélectionné.

10. Procédé suivant la revendication 9, dans lequel ledit antigène présélectionné présentant un intérêt est codé sur un polynucléotide préalablement introduit dans ladite cellule.

11. Procédé suivant la revendication 9 ou 10, comprenant l'arrêt de l'induction dudit allèle négatif dominant d'un gène de réparation de mésappariement, afin de stabiliser ladite cellule.
